# EUROPÄISCHE PATENTSCHRIFT

(11) **EP 1 386 161 B1**
(45) Veröffentlichungstag und Bekanntmachung des Hinweises auf die Patenterteilung: **14.03.2007**
(21) Anmeldenummer: 02748700.8
(22) Anmeldetag: 13.05.2002
(51) Int. Cl.: G01N 33/573, C12N 9/12, C07K 16/40, C12N 15/85

(54) **SCREENINGVERFAHREN MIT PIM1-KINASE ODER PIM3-KINASE**
SCREENING METHOD USING PIM1-KINASE OR PIM3-KINASE
PROCEDE DE CRIBLAGE AU MOYEN DE PIM1-KINASE OU PIM3-KINASE

(30) Priorität: 11.05.2001 DE 10123055
(43) Veröffentlichungstag der Anmeldung: 04.02.2004
(73) Patentinhaber: Grünenthal GmbH, 52078 Aachen (DE)
(72) Erfinder: WEIHE, Eberhard, 35037 Marburg (DE); SCHÄFER, Martin, K.-H., 35041 Marburg (DE); GILLEN, Clemens, 52159 Roetgen (DE)
(74) Vertreter: Hiebl, Inge Elisabeth
(86) Internationale Anmeldenummer: PCT/EP2002/005234
(87) Internationale Veröffentlichungsnummer: WO 2002/093173

(56) Entgegenhaltungen:
- EP-A- 0 911 391
- WO-A-00/75667
- WO-A-98/41639
- WO-A-03/060130
- US-A- 6 143 540
- MARCHAND ET AL.: "Role of the Immune System in Chronic Pain" NATURE REVIEWS, Bd. 6, Juli 2005 (2005-07), Seiten 521-532,

## Beschreibung

Die Erfindung betrifft ein Verfahren zur Auffindung schmerzrelevanter Substanzen unter Verwendung der PIM-1- oder PIM-3-Kinase.

Zur Therapie von Schmerzen stehen unterschiedliche Arzneimittel zur Verfügung wie z.B. Acetylsalicylsäure, Paracetamol, Dipyrone, Tramadol, Morphin und Fentanyl; aber auch Substanzen wie Amitryptilin und Ketamin kommen zur Behandlung von Schmerzpatienten zum Einsatz. Trotz zunehmend verfeinerter Therapieschemata kann jedoch insbesondere bei chronischen Schmerzzuständen oft keine dauerhafte Verbesserung für die Patienten erzielt werden. Hierfür ist unter anderem auch die Tatsache verantwortlich, daß es beim chronischen Schmerz zu dauerhaften Veränderungen beteiligter Nervenzellen kommt.

Die Schmerzforschung der letzten Jahre erbrachte die grundlegende Erkenntnis, daß der Entwicklung gerade chronischer Schmerzzustände plastische Veränderungen des Nervensystems, insbesondere in den nozizeptiven Neuronen der Hintervuurzelganglien und der Neurone im Bereich der Dorsalhömer des Rückenmarks, zugrunde liegen (als Überblick siehe: Coderre et al. 1993 ; Zimmermann & Herdegen, 1996). Die neuronale Plastizität geht einher mit Veränderungen in der Expression bestimmter Gene und führt zur langanhaltenden Veränderung des Phänotyps der betroffenen Neuronen. Das Konzept der neuronalen Plastizität wurde bisher vor allem auf Entwicklungs-, Lern-und Regenerationsprozesse angewandt, doch die neueren Befunde aus der Schmerzforschung zeigen, daß dieses Konzept auch bei pathophysiologischen Vorgängen greift (Tölle, 1997).

Die Chronifizierung des Schmerzes ist tierexperimentell auf phänomenologischer Ebene bereits relativ gut charakterisiert. Die Induktion chronischer Schmerzzustände führt zu folgenden Veränderungen:
- Erhöhte Empfindlichkeit und verringerte Reizschwelle peripherer Nozizeptoren
- Aktivierung sogenannter stiller Nozizeptoren
- Reorganisation rezeptiver Felder
- Erregbarkeitszunahme im Rückenmark.

Diese plastischen Veränderungen sind sowohl für die in den Ganglien vorkommenden primären Afferenzen, als auch für die im Rückenmark lokalisierten nachgeschalteten Neurone beschrieben worden und werden auch supraspinal z. B. im Thalamus vermutet. In Analogie zu den für Lern-und Gedächtnisprozesse beschriebenen Mechanismen ist anzunehmen, daß in den beteiligten Zellen ein spezifisches Genprogramm abläuft, das die koordinierte Regulation relevanter Gene beinhaltet, deren Expression dann maßgeblich zur pathophysiologischen Ausprägung chronischer Schmerzen beiträgt.

Ausgangspunkt der Erfindung war daher die Identifizierung derartiger schmerzregulierter Gene, die in ihrer Expression unter Schmerzbedingungen verändert und deshalb wahrscheinlich an der Entstehung und Verarbeitung von insbesondere chronischen Schmerzen beteiligt sind, über ihre Regulationszusammenhänge.

Für eine Reihe bekannter Gene wurde bereits eine Regulation in verschiedenen Schmerzmodellen nachgewiesen (s. Tabelle 1), so zum Beispiel für Neurotransmitter (Substanz P, CGRP), Rezeptoren (Substanz P-Rezeptor, µ, κ, δ-Opiatrezeptoren, NMDA-Rezeptor) und Transkriptionsfaktoren (cJun, JunB, cFos oder Krox24). Die Tatsache, daß die genannten Rezeptoren bereits als molekulare Targets für die Entwicklung neuer Analgetika verwendet werden (Dickenson, 1995), gibt einen deutlichen Hinweis darauf, daß auch die Identifizierung neuer schmerzregulierter Gene für die Entwicklung von Analgetika, insbesondere für entsprechende Screeningverfahren, von großem Interesse ist. Die zentrale Idee ist hierbei, die Entstehung oder Persistenz von Schmerzen, insbesondere chronischer Art, zu unterbrechen, indem solche Proteine in ihrer Funktion beeinflußt werden, die in Schmerz-Zuständen verstärkt oder vermindert gebildet werden.

**Tabelle 1: Regulation bekannter Gene/Genprodukte in Schmerz-Tiermodellen**

| **Gen(produkt)** | **Reg** | **Gewebe/Zelle** | **Modell** | **Literatur** |
|---|---|---|---|---|
| **(a) Neurotransmitter** | | | | |
| CGRP | ⇑ | RM-Dorsalhorn | UV-Bestrahlung | Gillardon F et al. (1992) |
| | | | der Haut | Ann NY Acad Sci657: 493- |
| | | | | 96 |
| Preprotachykinin & | ⇑ | DRG | Monoarthritis | Donaldson LF et al. (1992) |
| CGRP-mRNA | | | | Mol Brain Res 16: 143-49 |
| Preprotachykinin | ⇑ | RM-Dorsalhorn | Formalin | Noguchi & Ruda (1992) |
| -mRNA | | | | J Neurosci 12:2563-72 |
| Prodynorphin mRNA | ⇑ | Rückenmark | Exp Arthritis | Höllt et al (1987) |
| | | | | Neurosci Lett 96:247-52 |
| Dynorphin Prot. | ⇑ | Rückenmark | Formalin | Ruda et al. (1988) |
| | | | | PNAS 85: 622-26 |
| Substanz P | ⇑ | Nozizeptoren | Exp. Arthritis | Levine JD et al. (1984) |
| | | | | Science 226:547-49 |
| **(b) Neurotrophine** | | | | |
| BDNF mRNA & | ⇑ | DRG: trkA+ | i. th. NGF Inj. | Michael GC et al. (1997) |
| Immunreaktivität | | Zellen | | J Neurosci 17: 8476-90 |
| **(c) Rezeptoren** | | | | |
| µ, κ, δ-Bindung | ⇓ ⇑ | RM-Dorsalhom | Monoarthritis | Besse D et al. (1992) |
| | | | | Eur J Pharmacol 223:123- |
| | | | | 31 |
| µ-Opiatrezeptor- | ⇑ | DRG | Carageenan ind. | Ji R-R et al. (1995) |
| lmmunreaktivität | | | Entzündung | J Neurosci 15: 8156-66 |
| κ & δ-Opiatrez.-mRNA | ⇓ | DRG | Carageenan ind. | Ji R-R et al. (1995) |
| | | | Entzündung | J Neurosci 15: 8156-66 |
| κ & µ-Opiatrezeptor-mRNA | ⇑ | RM-Dorsalhom | Monoarthritis | Maekawa K et al. (1995) |
| | | | | Pain 64:365-71 |
| CCK_{B}-Rez. mRNA | ⇑ | DRG | Axotomie | Zhang X et al. (1993) |
| | | | | Neuroscience 57: 227-233 |
| NMDA-R1-mRNA | ⇓ | RM-Dorsalhom | CFA-induzierte | Kus L et al. (1995) |
| | | Laminae 1 & 11 | Entzündung | Neuroscience 68:159-65 |
| **(d) Enzyme** | | | | |
| NADPH-Diaphorase | ⇑ | RM-Dorsalhom | Ischiaticus- | Fiallos-Estrada et al. (93) |
| Aktivität | | | Transektion | Neurosci Lett 150: 169-73 |
| NADPH-Diaphorase | ⇑ | Rückenmark | Formalin | Solodkin et al. 1992 |
| | | | | Neurosci 51: 495-99 |
| NO-Synthetase mRNA | ⇑ | DRG | Axotomie | Verge VMK et al. (1992) |
| | | | | PNAS 89: 11617-62 |
| NO-Synthetase Protein | ⇑ | RM-Dorsalhom | Formalin | Herdegen et al. (1994) |
| | | | | Mol Brain Res 22:245-58 |
| NO-Synthetase- | ⇑ | DRG | lschiaticus- | Fiallos-Estrada et al. ('93) |
| Immunreaktivität | | | Transektion | Neurosci Lett 150: 169-73 |
| **(e) Signalkaskade** | | | | |
| rap1A, rap1B, H-ras | ⇑ | Rückenmark | Formalin | Urayama O et al. (1997) |
| mRNA | | | | Mol Brain Res 45:331-34 |
| PKC-Bindung | ⇑ | RM-Dorsalhom | CFA-induzierte | Tölle TR et al (82) |
| | | | Monoarthritis | J Neurol 242(S2):135 |
| **(f)Transkriptionsf.** | | | | |
| cFOS | ⇑ | Rückenmark | Noxische | Hunt SP et al. (1987) |
| | | | Stimulierung | Nature 328: 632-34 |
| cJun,JunB, cFOS | ⇑ | RM-Dorsalhom | Formalin | Herdegen T et al. (1994) |
| Kro×24 | | | | Mol Brain Res 22: 245-48 |

| | | | | |
|---|---|---|---|---|
| RM, Rückenmark : DRG, Dorsal Root Ganglia; CFA, Complete Freund Adjunvans; NGF, Nerve Growth Factor. | | | | |

Zudem beschreibt US 6,143,540 HKID-1 Polypeptide, Proteine und dafür kodierende Nukleinsäuremoleküle. Diese HKID-1 Polypeptide, Proteine weisen eine 77%-ige Ähnlichkeit zu PIM-1- aus Xenopus laevis auf. Zwar offenbart US 6,143,540 auch diagnostische Verfahren, Screeningverfahren und therapeutische Verfahren unter Verwendung der dort genannten Zusammensetzungen, jedoch werden dort keine Zusammenhänge mit Schmerz oder schmerzregulierende Substanzen beschrieben.

WO 00/75667 offenbart Verfahren zur Untersuchung von Verbindungen, die die Phosphorylierung, insbesondere einer Phosphatase oder Proteinkinase in einer Reaktionsmischung modulieren. WO 00/75667 beschreibt jedoch weder PIM-1- oder PIM-3-Kinasen noch Verfahren zur Identifizierung schmerzregulierender Substanzen.

EP-A-0 911 391 offenbart HWHHJ20 Polypeptide und Verfahren zur Herstellung solcher Polypeptide durch rekombinante Verfahren. Weiterhin offenbart EP-A-0 911 391 Verfahren zur Verwendung dieser Polypeptide sowie dafür kodierender Polynukleotide in der Therapie und in diagnostischen Assays. Allerdings beschreibt auch EP-A-0 911 391 weder P1M-1- oder PIM-3-Kinasen noch Verfahren zur Identifizierung schmerzregulierender Substanzen.

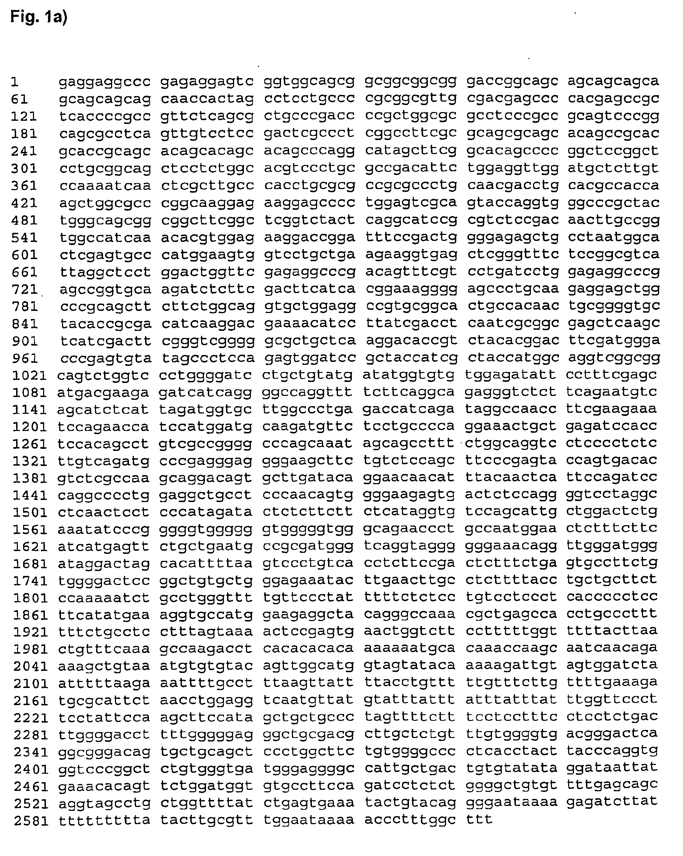

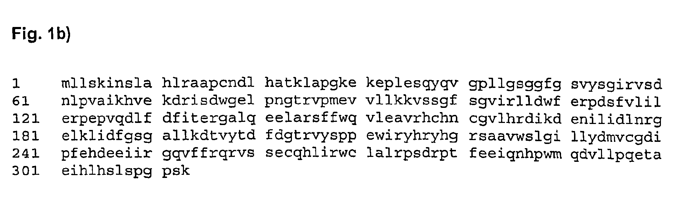

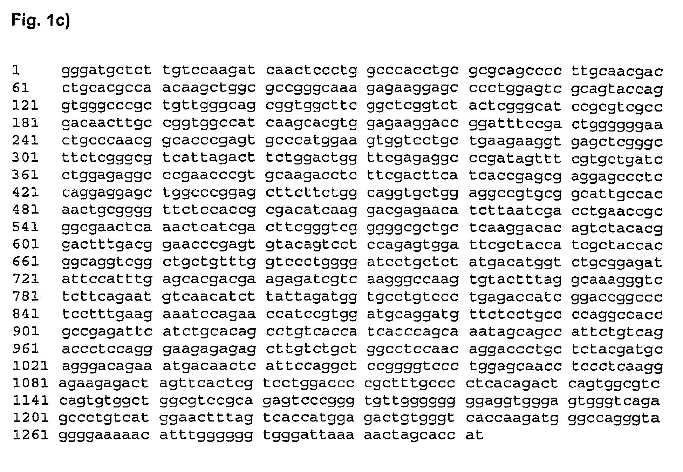

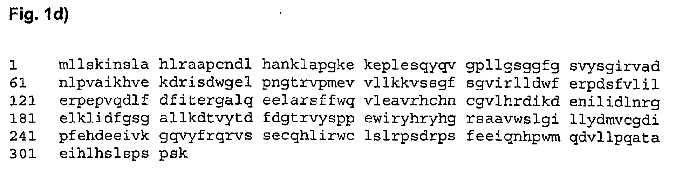

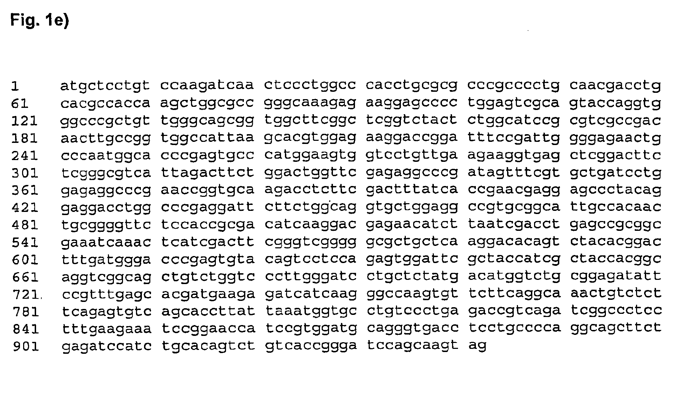

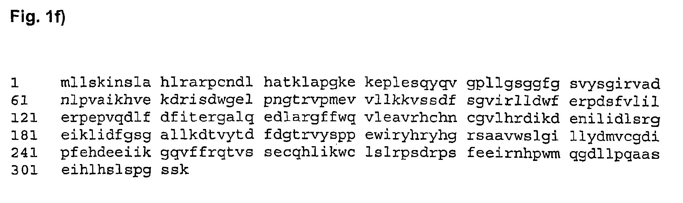

Daraus folgend war primäre Aufgabe der Erfindung, ein Screeningverfahren zur Identifizierung im Schmerz relevanter, insbesondere schmerzregulierender Substanzen zu entwickeln. Die Erfindung betrifft daher ein Verfahren zur Auffindung schmerzregulierender Substanzen mit folgenden Verfahrensschritten:
(a) Inkubation einer zu testenden Substanz unter geeigneten Bedingungen mit einer Zelle und/oder einer Präparation aus einer solchen Zelle, die das Protein PIM-1-Kinase oder PIM-3-Kinase und/oder ein Protein gemäß einer der Abbildungen 1b), 1d), 1f), 2d) oder 2f) und/oder ein zu einem dieser vorgenannten Proteine zu mindestens 90 % ähnliches Protein und/oder ein Protein, für das ein Polynukleotid gemäß einer der Abbildungen 1a), 1c), 1e), 2a), 2c) oder 2e) oder ein dazu zu mindestens 90 % ähnliches Polynukleotid kodiert, und/oder ein Protein, das durch eine Nukleinsäure kodiert wird, die unter stringenten Bedingungen an ein Polynukleotid gemäß einer der Abbildungen 1a), 1c), 1e), 2a), 2c) oder 2e) oder deren Antisense Polynukleotide binden,
(b) Messung der Bindung der Testsubstanz an dem von der Zelle synthetisierten Protein oder Messung mindestens eines der durch die Bindung der Testsubstanz an das Protein veränderten funktionellen Parameter
   über Messung der Regulation, Hemmung und/oder Aktivierung von Rezeptoren, lonenkanälen und/oder Enzymen, insbesondere über Messung der Veränderung der Genexpression, des lonenmileus, des pH oder des Membranpotentials, über Veränderung der Enzymaktivität oder der Konzentration der 2^{nd} messenger, oder
   über Messung der Bindung über die Verdrängung eines bekannten markierten Liganden des Proteins und/oder über die daran gebundene Aktivität einer markierten Testsubstanz.

Dieses neuartige Screeningverfahren basiert darauf, daß hier eine potentielle Schmerz-Wirksamkeit einer Substanz über ihre Wechselwirkung mit einer schmerzregulierten Proteinstruktur, insbesondere PIM1-Kinase oder verwandte Strukturen, oder über eine Proteinstruktur mit einer schmerzrelevanten Verteilung im ZNS, insbesondere P1M3-Kinase oder verwandte Strukturen, aufgefunden werden kann.

Dabei bezieht sich der Begriff schmerzregulierend auf einen potentiellen regulierenden Einfluß auf das physiologische Schmerzgeschehen, insbesondere auf eine analgetische Wirkung. Der Begriff Substanz umfaßt jede als Arzneimittel-Wirkstoff geeignete Verbindung, insbesondere also niedermolekulare Wirkstoffe, aber auch andere wie Nukleinsäuren, Fette, Zucker, Peptide oder Proteine wie Antikörper.

Die Inkubation unter geeigneten Bedingungen ist hier so zu verstehen, daß die zu untersuchende Substanz mit der Zelle oder der entsprechenden Präparation in einem wässrigen Medium eine definierte Zeit vor der Messung reagieren kann. Dabei kann das wässrige Medium temperiert werden, beispielsweise zwischen 4°C und 40°C, vorzugsweise bei Raumtemperatur oder bei 37°C. Die Inkubationszeit kann zwischen wenigen Sekunden und mehreren Stunden variiert werden, je nach der Wechselwirkung der Substanz mit dem Protein. Bevorzugt sind aber Zeiten zwischen 1 min und 60 min. Das wäßrige Medium kann geeignete Salze und/oder Puffersysteme enthalten, so daß bei der Inkubation beispielsweise ein pH zwischen 6 und 8, vorzugsweise pH 7,0 - 7,5 im Medium herrscht. Dem Medium können weiter geeignete Substanzen, wie Coenzyme, Nährstoffe etc. beigefügt werden. Die geeigneten Bedingungen können vom Fachmann in Abhängigkeit von der zu untersuchenden Wechselwirkung der Substanz mit dem Protein aufgrund seiner Erfahrung, der Literatur oder weniger, einfacher Vorversuche leicht festgelegt werden, um im Verfahren einen möglichst deutlichen Meßwert zu erhalten.

Eine Zelle, die ein bestimmtes Protein synthetisiert hat, ist eine Zelle, die dieses Protein bereits endogen exprimiert hat oder eine solche, die gentechnisch verändert wurden, so daß sie dieses Protein exprimiert und entsprechend vor Beginn des erfindungsgemäßen Verfahrens das Protein enthält. Die Zellen können Zellen aus evt. immortalisierten Zellinien sein oder native aus Geweben stammende und aus diesen isolierte Zellen sein, wobei der Zellverband meist aufgelöst ist. Die Präparation aus diesen Zellen umfaßt insbesondere Homogenate aus den Zellen, das Cytosol, eine Membranfraktion der Zellen mit Membranfragmenten, eine Suspension isolierter Zellorganellen etc.

Die hier aufgezählten Proteine wurden im Rahmen dieser Erfindung als durch Schmerz reguliert oder schmerzrelevant verteilt identifiziert, in dem in einem Tier Schmerz ausgelöst wurde und nach angemessener Zeit durch Schnitte im Rückenmark das Expressionsmuster des Tieres mit denen eines Kontroll-Tieres ohne schmerzauslösende Maßnahmen verglichen. Die dabei gefundenen verändert exprimierten sind die PIM-1-Kinase sowie insbesondere bezüglich der schmerzrelevanten Verteilung die PIM-3-Kinase.

Aus welcher Spezies diese Proteine stammen, ist für die Funktion des Verfahrens unerheblich, es ist aber bevorzugt, die humane, Maus- oder Ratten-Variante zu verwenden. Die PIM1-Kinase ist in Hinblick auf die kodierende DNA- und die Aminosäure-Sequenz bekannt und auch in Ihrer generellen Funktion beschrieben. Das trifft zum Teil auch auf die PIM3-Kinase zu, wobei bei dieser die bisher noch nicht bekannte kodierende DNA- und die Aminosäure-Sequenz in Maus und Mensch im Rahmen dieser Erfindung aufgeklärt wurde. Keine dieser Kinasen wurde aber bisher im Stand der Technik in einen Zusammenhang mit Schmerz und insbesondere der Schmerzregulation gebracht. Da hier die Identifizierung der Proteine über eine Veränderung der Expression oder die Expressionsverteilung in einem In-vivo-Schmerzmodell erfolgte, hat das daraus abgeleitete erfindungsgemäße Screening-Verfahren für zukünftige Arzneimittel unter Verwendung dieser Proteine den erheblichen Vorteil, nicht nur auf theoretischen Überlegungen aufzubauen, sondern vermutlich eine starke In-vivo-Relevanz zu besitzen. Da mit diesem Verfahren die Wechselwirkung von Substanzen mit im Schmerzbereich bisher nicht verwendeten Proteinen und Peptiden als Maßstab für das Auffinden schmerzregulierender Substanzen ermöglicht wird, sind mit diesem Verfahren jetzt möglicherweise schmerzrelevante Substanzen aufzufinden, die bei den im Stand der Technik bisher bekannten Verfahren mit anderen Peptiden oder Proteinen nicht aufgefallen wären. Auch dies ist ein erheblicher Vorteil des neuen erfindungsgemäßen Verfahrens.

Der Maßstab über den das Verfahren die Auffindung interessanter Substanzen erlaubt, ist entweder die Bindung an das Protein ,die z.B. durch Verdrängung eines bekannten Liganden oder das Ausmaß gebundener Substanz nachgewiesen werden kann, oder die Veränderung eines funktionellen Parameters durch die Wechselwirkung der Substanz mit dem Protein. Diese Wechselwirkung kann insbesondere in einer Regulation, Hemmung und/oder Aktivierung von Rezeptoren, Ionenkanälen und/oder Enzymen liegen und veränderte funktionelle Parameter können beispielsweise die Genexpression, das Ionenmilieus, der pH oder das Membranpotentials, bzw. die Veränderung der Enzymaktivität oder der Konzentration der 2^{nd} messenger sein.

Zur Erläuterung der Erfindung werden im folgenden neben den im allgemeinen Text zu Begriffen gegebenen Erklärungen weitere Definitionen angegeben, um klarzustellen, wie bestimmte, insbesondere in den Ansprüchen verwendete Begriffe im Sinne dieser Erfindung zu verstehen und auszulegen sind.
- Substanz: Damit ist eine chemische Verbindung gemeint. Hier handelt es sich im engeren Sinne um Verbindungen, die potentiell eine Wirkung im Körper entfalten können, niedermolekulare Wirkstoffe, Nukleinsäuren, Fette, Zucker, Peptide oder Proteine, insbesondere hier niedermolekulare Wirkstoffe.
- schmerzregulierend: Im Sinne der Erfindung heißt schmerzregulierend, daß die Substanz die Wahmehmung von Schmerz direkt oder indirekt beeinflußt, insbesondere natürlich analgetisch wirkt.
- Inkubation: Unter Inkubation ist das Einbringen und Belassen eines biologischen Untersuchungsobjektes, beispielsweise einer Zelle oder eines Proteins, in einem temperierten Medium wie in einem Brutschrank oder auf einem Wasserbad zu verstehen. Dabei heißt hier unter geeigneten Bedingungen eine Inkubation unter physiologischen Bedingungen (z.B. 37°C pH7,2) oder bei den Bedingungen, bei denen eine optimale Messung im Verfahren möglich wird.
- Zelle: Die Zelle ist ein sich selbst regulierendes, offenes, mit seiner Umgebung durch permanenten Stoffaustausch in einem Fließgleichgewicht stehendes System mit eigenem Stoffwechsel, und Vermehrungsfähigkeit Die Zelle kann separat kultiviert oder Teil eines Gewebes, insbesondere aus einem Organ, sein, und dort vereinzelt oder noch im Zellverband vorliegen.
- Präparation aus einer Zelle: Darunter versteht man Präparate, die mittels chemischer, biologischer, mechanischer oder physikalischer Methoden unter Änderung der Zellstruktur hergestellt werden, beispielsweise Membranfragmente, Isolierte Zellkompartimerite, isoliertes Cytosol, oder aus Gewebe gewonnenes Homogenat.
- Peptid: Verbindung aus über peptidische Bindungen zu Ketten verknüpften Aminosäuren. Ein Oligopeptid besteht aus zwischen 2 und 9 Aminosäuren, ein Polypeptid aus zwischen 10 und 100 Aminosäuren.
- Protein: Verbindung aus über peptidische Bindungen zu Ketten verknüpften mehr als 100 Aminosäuren u.U. mit einer definierten Raumstruktur.
- PIM1-Kinase; PIM3-Kinase: Ein Proto-Oncogen und eine Serin-Threonin-Kinase.
- Polynukleotid: Das zugrundeliegende Nukleotid ist ein grundsätzlich aus Nucleinbase, Pentose und Phosphorsäure bestehender Grundbaustein der Nucleinsäuren. Diese entspricht einem hochmolekularen Polynucleotid aus mehreren Nukleotiden, die über Phosphorsäure-Pentose-Veresterung miteinander verknüpft sind. Unter dieser Erfindung fallen aber auch modifizierte Polynukleotide, die zwar die Basenabfolge beibehalten, aber über ein modifiziertes Rückgrat statt der Phosphorsäure-Pentose verfügen.
- zu mindestens 90 (95, 97)% ähnlich: Darunter ist zu verstehen, daß die mit erfaßten Polynucleotide in ihrem kodierenden Bereich bezüglich der Basenabfolge zu mindestens 90% (95%, 97%) identisch mit der Referenz (Abbildung etc.) sind und die mit erfaßten Peptide und Proteine in ihrer Primärstruktur, der Abfolge der Aminosäuren zu mindestens 90% (95%, 97%) mit der Referenz identisch sind.
- Gen: Mit dem Begriff Gen wird ein Genomabschnitt mit einer definierten Nukleotidsequenz bezeichnet, der die Information zur Synthese einer m- oder prä-mRNA oder einer sonstigen RNA (z.B. tRNA, rRNA, snRNA etc.) enthält. Es besteht aus kodierenden und nicht kodierenden Abschnitten.
- Genfragment: Nukleinsäureabschnitt, der in seiner Basenabfolge einen Teilbereich eines Gens beinhaltet
- Bindung an das Peptid Protein: Wechselwirkung zwischen Substanz und Peptid Protein, die zu Fixierung führt.
- funktionelle Parameter: darunter versteht man Meßgrößen eines Experimentes, die mit der Funktion eines Proteins (Ionenkanal, Rezeptor, Enzym) korrelieren
- gentechnisch manipuliert: Manipulation von Zellen, Geweben oder Organismen derart, daß hier genetisches Material eingebracht wird
- endogen exprimiert: Expression eines Proteins, die eine Zelllinle unter geeigneten Kulturbedingungen aufweist, ohne dass dieses entsprechende Protein durch gentechnische Manipulation zur Expression veranlasst wurde
- G-Protein: International Übliche Abkürzung für ein Guanosintriphosphat (GTP)-bindendes Protein, das als Signalprotein durch G-Protein gekoppelte Rezeptoren aktiviert wird
- Reportergen: Generelle Bezeichnung für Gene, deren Genprodukte sich mit Hilfe einfacher biochemischer Methoden oder histochemischer Methoden einfach nachweisen lassen, wie z.B. Luziferase, alkalische Phosphatase oder Green Fluorescent Protein (GFP).
- (rekombinantes) DNA-Konstrukt: Generelle Bezeichnung für jede Art von DNA-Molekülen, die durch die in vitro-Verknüpfung von DNA-Molekülen entstanden sind.
- Klonierungsvektor: Generelle Bezeichnung für Nukleinsäure-Moleküle, die beim Klonieren als Träger von Fremdgenen oder Teilen dieser Gene dienen.
- Expressionsvektor: Bezeichnung für speziell konstruierte Klonierungsvektoren, die nach Einbringen in eine geeignete Wirtszelle die Transcription und Translation des in den Vektor einklonierten Fremdgens erlauben.
- LTR-Sequenz: Abkürzung für Long terminal repeat. Generelle Bezeichnung für längere Sequenzbereiche, die an beiden Enden eines linearen Genoms zu finden sind. Derartige Sequenzbereiche kommen z.B. in den Genomen von Retroviren und an den Enden eukaryotischer Transposons vor.
- Poly-A-Schwanz: die am 3'-Ende von messenger-RNAs durch Polyadenylierung angeheftenen Adenyl-Reste (ca. 20-250).
- Promotor-Sequenz: Bezeichnung für einen DNA-Sequenzbereich, von dem aus die Transkription eines Gens, d.h. die Synthese der mRNA, gesteuert wird.
- ORI-Sequenz : Abkürzung für Origin of replication. Die ORI-Sequenz erlaubt einem DNA-Molekül, sich als autonome Einheit in der Zelle zu vermehren.
- Enhancer-Sequenz: Bezeichung für relativ kurze, zum Teil als Repetitionen auftretende, genetische Elemente, die in der Regel die Expression mancher Gene in unterschiedlichem Maße verstärken.
- Transkriptionsfaktor: Bezeichnung für ein Protein, das über eine Bindung an spezifische DNA-Sequenzen, die Transkription eines Gens beeinflußt.
- kultivieren: Zellen oder Gewebe unter geeigneten Kulturbedingungen halten
- Bedingungen, die eine Expression erlauben, darunter versteht man die Auswahl und Anwendung von Kulturbedingungen die eine Expression des interessierenden Proteins erlauben, darunter gehören Temperaturänderung, Mediumwechsel, Zusatz von induzierenden Substanzen, Weglassen hemmender Substanzen.
- Inkubationszeit: Zeitdauer für die Zellen oder Gewebe inkubiert, d.h. einer definierten Temperatur ausgesetzt werden.
- Selektionsdruck**:** Anwendungen von Kulturbedingungen die Zellen mit einem bestimmtem Genprodukt, dem sog. Selektionsmarker, einen Wachstumsvorteil verschaffen.
- Amphibienzelle, Zelle aus einem Tier der Klasse der Amphibia
- Bakterienzelle, Zelle die dem Überreich der Eubacteria oder Archaebacteria zuzuordnen ist, oder von ihr abstammt.
- Hefezelle, Zelle die der Ordnung der Endomycetalse zuzuordnen ist, oder von ihr abstammt.
- Insektenzelle, Zelle die der Ordnung der Hexapoda zuzuordnen ist, oder von ihr abstammt.
- native Säugetierzelle, aus einem Säugetier stammende Zelle, die in ihren relevanten Merkmalen der im Organismus befindlichen Zelle entspricht.
- immortalisierte Säugetierzelle : Zelle die durch die angewendeten Kulturbedingungen oder gentechnische Manipulation die Eigenschaft erlangt hat, sich über die normalerweise übliche Teilungshäufigkeit hinaus (ca.100) in der Kultur zu teilen.
- markiert : durch entsprechende Modifizierung oder Derivatisierung für eine Nachweisreaktion zugänglich gemacht. Beispielsweise radioaktiv, fluoreszierend oder lumineszierend.
- Ligand: Substanz, die an ein im Körper oder einer Zelle befindliches Molekül, im speziellen einen Rezeptor, bindet
- Verdrängung: vollständiges oder partielles Entfernen eines Liganden von seiner Bindungsstelle
- gebundene Aktivität: Biochemisch oder physikalisch erfaßter Meßwert, der mit der an einem Rezptor gebundenen Ligandenmenge korreliert
- Regulation: die als Teil eines Regelprozesses erfolgte Hemmung oder Aktivierung eines Vorgangs
- Hemmung : als Sonderfall der Regulation die Verhinderung/Minderung eines Vorgangs
- Aktivierung: als Sonderfall der Regulation die Verstärkung eines Vorgangs
- Rezeptoren, Im weitesten Sinne alle im pro- oder eukaryoten Organismus vorhandenen Moleküle, an die ein Wirkstoff binden kann. Im engeren Sinne membrangebundene Proteine oder Komplexe mehrerer Proteine, die durch Bindung eines Wirkstoffes eine Änderung in der Zelle hervorrufen.
- Ionenkanäle: Membrangebundene Proteine oder Komplexe mehrerer Proteine, durch die Kationen oder Anionen durch die Membran hindurchgelangen können.
- Enzyme: Bezeichnung für Proteine oder Komplexe aus einer aktivierenden Nichteiweißkomponente mit einem Protein, die katalytische Eigenschaften besitzen.
- Genexpression (exprimieren/exprimierbar): das Übersetzen der genetischen Information eines Genes in RNA (RNA-Expression) oder in Protein (Proteinexpression).
- Ionenmilieu: lonenkonzentration eines oder mehrerer Ionen in einem bestimmten Kompartiment.
- Membranpotential: Spannungsdifferenz über eine Membran aufgrund eines Überschusses an Kationen auf der einen Seite und Anionen auf der anderen Seite der Membran.
- Veränderung der Enzymaktivität: Hemmung oder Induktion der katalytischen Aktivität eines Enzyms.
- 2^{nd} messenger: kleines Molekül, das als Antwort auf ein extrazelluläres Signal entweder im Cytosol gebildet wird oder in das Cytosol hineinwandert und dabei hilft die Information an das Zellinnere weiterzugeben, wie zum Beispiel cAMP, IP₃.
- (Gen-)Sonde: Bezeichnung für jede Art von Nukleinsäuren, mit deren Hilfe man ein gesuchtes Gen oder eine bestimmte DNA-Sequenz nachweisen kann. Durch Derivatisierung der Gensonde (z.B. Biotin, magnetische Beads, Digoxinin) können zudem DNA-Moleküle aus einem Gemisch herausgezogen werden. Als Sonden werden klonierte Gene, Genfragmente, chemisch synthetisierte Oligonukleotide und auch RNA verwendet, die meist radioaktiv markiert ist.
- DNA: Internationale Bezeichnung für Desoxyribonukleinsäure
- genomische DNA: Generelle Bezeichnung für die bei eukaryotischen Organismen aus dem Zellkern einer Zelle stammenden DNA.
- cDNA: Abkürzung für complementary DNA. Bezeichnung für die einzel- bzw. doppelsträngige DNA-Kopie eines RNA-Moleküls.
- cDNA-Bank/Bibliothek: Bezeichung für eine Sammlung von willkürlich klonierten cDNA-Fragmenten, die zusammengenommen die Gesamtheit aller von einer Zelle oder einem Gewebe synthetisierten RNA repäsentieren.
- cDNA-Klon : Bezeichnung für eine Population genetisch einheitlicher Zellen, die sich von einer einzigen Zelle ableiten, derart, daß diese Zelle eine künstlich eingebrachtes cDNA-Fragment enthält.
- Hybridisierung: Durch Basenpaarung bewirkte Ausbildung eines doppelsträngigen Nukleinsäuremoleküls aus zwei getrennten Einzelsträngen.
- stringente Bedingungen : Bedingungen, unter denen nur perfekt basengepaarte Nukleinsäure-Stränge gebildet werden und stabil bleiben.
- isolieren: ein gesuchtes Molekül aus einem Gemisch herausfinden und abtrennen.
- DNA-Sequenzierung: Bestimmung der Abfolge von Basen in einem DNA-Molekül.
- Nukleinsäuresequenz: Bezeichnung für die Primärstruktur eines DNA-Moleküls, d.h. die Abfolge der einzelnen Basen, aus denen sich eine DNA zusammensetzt.
- Genspezifische Oligonukleotid Primer: Oligonukleinsäuren, also 10-40 Basen lange Nukleinsäurefragmente, die in ihrer Basenzusammensetzung eine stringente Hybridisierung an das gesuchte Gen oder die gesuchte cDNA erlauben.
- Ermitteln von Oligonukleotid Primern: Die manuelle oder Computerunterstützte Suche von Oligonukleotiden zu einer vorgegebenen DNA-Sequenz, die für eine Hybridisierung und/oder eine Polymerase-Ketten Reaktion optimal geeignet sind.
- PCR: Abkürzung für Polymerase-Kettenreaktion. In vitro-Verfahren zur selektiven Anreicherung von Nucleinsäure-Bereichen definierter Länge und definierter Sequenz aus einem Gemisch von NukleinsäureMolekülen.
- DNA-Template: Nukleinsäuremolekül oder ein Gemisch von Nukleinsäuremolekülen, aus denen ein DNA-Abschnitt mit Hilfe der PCR (s.o.) vervielfältigt wird.
- RNA: International gebräuchliche Abkürzung für Ribonukleinsäuren
- mRNA: International gebräuchliche Abkürzung für messenger-Ribonukleinsäuren, die am Transfer der genetischen Information aus dem Kern in die Zelle beteiligt sind und die Information für die Synthese eines Polypetids oder eines Proteins beinhalten.
- Antisense Polynukleotid: Eine aus mehreren natürlichen oder modifizierten Nukleinsäuren bestehendes Molekül, deren Basenabfolge komplementär zur Basenabfolge eines Teilbereiches einer in der Natur vorkommenden RNA ist.
- PNA: International gebräuchliche Abkürzung für peptidic Nucleic Acids. Hierbei bilden peptidisch verknüpfte Aminosäuren eine Kette, wobei die Aminosäuren als Seitenkette eine für die Hybridisierung mit DNA oder RNA fähigen Base trägt.
- Sequenz : Abfolge von Nukleotiden oder Aminosäuren. Im spezifischen Sinne dieser Erfindung ist damit die Nukleinsäuresequenz gemeint.
- Ribozym: Bezeichnung für eine katalytisch aktive Ribonukleinsäure (z.B. Ligase, Endonuklease, Polymerase, Exonuklease)
- DNA-Enzym: Bezeichnung für ein DNA-Molekül, das katalytische Aktivität beinhaltet (z.B. Ligase, Endonuklease, Polymerase, Exonuklease)
- katalytische RNA/DNA : generelle Bezeichnung für Ribozyme bzw. DNA-Enzyme (s.o.).
- Adenovirus: bei Vertebraten vorkommender cytopathogener Virus
- Adenoassoziiertes Virus (AAV) : Gehört zur Familie der Parvoviren. Für eine effektive Vermehrung des AAV ist eine Coinfektion der Wirtszellen mit Helferviren (z.B. Herpes-, Vaccina- oder Adenoviren) erforderlich. Die Eigenschaft von AAV, stabil in das Wirtsgenom zu integrieren, macht es als Transduktionsvektor für Säugetierzellen besonders interessant.
- Herpesvirus : Viraler Erreger der Herpes-Infektion
- posttranslationale Modifikation: Veränderung an Proteinen oder Polypetiden, die nach der Translation durchgeführt wird, hierzu zählen z.B. Phosphorylierung, Glykosylierung, Amidierung, Acetylierung oder Proteolyse.
- glykosylieren Bezeichnung für das Anhängen von einzelnen Zuckermolekülen oder ganzen Zuckerketten an Proteine.
- phosphorylieren : Bezeichnung für das Anhängen von einem oder mehreren Phosphatresten an ein Protein, bevorzugt an die OH-Gruppen der Aminosäuren Serin, Threonin oder Tyrosin.
- amidieren. Die Bezeichnung für das Umwandeln einer Carboxylfunktion in eine Amidfunktion, z.B. an den carboxyterminalen Aminosäurerest eines Peptides oder Proteins.
- mit Membrananker versehen: Posttranslationelle Modifikation eines Proteins, oder eines anderen organischen Moleküls, derart daß es durch Anhängen eines hydrophoben Moleküls, geeigneterweise eine Fettsäure oder ein Derivat derselben, an die Lipiddoppelschicht-Membran von Zellen verankert wird.
- spalten: in diesem spezifischen Fall die Spaltung eines Peptids oder Proteins in mehrere Untersequenzen.
- verkürzen: Ein aus mehreren Einzelteilen bestehendes Molekül um eine oder mehrere Teile zu verkürzen.
- Antikörper: Lösliche, oder an Zellmembranen gebundene, als Immunglobuline bezeichnete Proteine mit einer spezifischen Bindungsstelle für Antigene.
- monoklonaler Antikörper: sind gegen eine einzige antigene Determinante eines Antigens gerichtete Antikörper mit extrem hoher Selektivität
- polyklonaler Antikörper: Gemisch aus Antikörpern, die gegen mehrere Determinanten eines Antigens gerichtet sind.
- transgen: genetisch verändert
- nichthumanes Säugetier: Die Gesamtheit der Säugetiere (Klasse der Mammalia) mit Ausnahme der Spezies Mensch.
- Keim-Zelle: Zelle mit haploidem Genom, die durch Verschmelzung mit einer zweiten Keimzelle die Bildung eines neuen Organismus ermöglicht.
- somatische Zelle: diploide Zelle als Bestandteil eines Organismus
- chromosomale Einbringung: Eingriff In die Nukleotidsequenz auf chromosomaler Ebene
- Genom: Allgemeine Beschreibung für die Gesamtheit aller Gene in einem Organismus
- Vorfahr des Tieres: Ein Tier (der Vorfahr), das auf natürliche oder künstliche Weise durch Weitergabe an seinem genetischen Material in direkter Linie mit einem anderen Tier (dem Nachfahren) verwandt ist.
- exprimierbar: Ein Nukleinsäuremolekül ist dann exprimierbar, wenn es die Information zur Synthese eines Proteins oder Polypetids beinhaltet und mit ensprechenden regulatorischen Sequenzen versehen ist, die eine Synthese dieses Proteins oder Polypeptids in vitro oder in vivo erlauben. Wenn diese Voraussetzungen nicht mehr gegeben sind, beispielsweise durch Eingriff in der kodierenden Sequenz, ist das Nukleinsäuremotekül nicht mehr exprimierbar.
- Nagetier: Tier aus der Ordnung der Rodentia, z.B. Ratte oder Maus
- als schmerzregulierende Substanz identifizierbar: Substanz die bei Einbringung in einen lebenden Organismus eine Verhaftensänderung bewirkt, die der Fachmann als schmerzhemmend bezeichnet (antinozizeptiv, antihyperalgetisch oder antiallodynisch). Im Falle des Screeningverfahrens bezieht sich das darauf, daß die Substanz beim Screening durch stärkere Bindung oder Auslösung einer Änderung eines funktionellen Parameters deutlich, beispielsweise 100 %, die Bindung oder Wechselwirkung des Durchschnitts der getesteten Substanzen übertrifft.
- Verbindung: ein anderer Name für Molekül, als aus mehreren Atomen bestehend, hier ein durch das erfindungsgemäße Verfahren identifiziertes Molekül.
- Wirkstoff: Eine Verbindung, die bei Anwendung an einem Organismus eine Veränderung in diesem Organismus hervorruft. Im Besonderen werden darunter organisch-chemisch synthetisierte Moleküle verstanden, die auf den Organismus eine heilende Wirkung ausüben. Hier insbesondere Moleküle, die an die erfindungsgemäß verwendeten, Proteine und Peptide binden.
- niedermolekular: Molekül mit einem Molekulargewicht < 2kDa
- Arzneimittel: ein Stoff entsprechend der Definition im Artikel 1 §2 des Gesetzes über den Verkehr mit Arzneimitteln
- Diagnostikum: Verbindung oder Verfahren, das verwendet werden kann, um eine Krankheit zu diagnostizieren
- Behandlung von Schmerz: Verfahren, mit dem Ziel Schmerzen zu lindern oder aufzuheben, oder das zu erwartende Auftreten von Schmerzen zu hemmen (preemptive Analgesie).
- chronischer Schmerz: eine Schmerzempfindung von länger anhaltender Dauer, oft dadurch gekennzeichnet, daß sie über Zeitpunkt und Ort des initialen Stimulus hinausreicht, die Schmerzempfindlichkeit des Körpers steigert.
- Gentherapie: Unter Gentherapie versteht man alle Verfahren, die das Ziel haben, genetische Erkrankungen durch geeignete Veränderungen des Genoms kausal zu behandeln.
- in-vivo-Gentherapie: Einbringen von genetischem Material in den lebenden Organismus mit dem Ziel der Gentherapie. Man kann zwischen somatischem und Keimbahn-Eingriff unterscheiden, der einmal an diploiden Zellen und einmal an haploiden Zellen stattfindet.
- In-vitro-Gentherapie: Einbringen von genetischem Material in Zellen außerhalb des menschlichen Körpers mit dem Ziel diese nachher wieder durch Einbringen in den menschlichen Körper zur Gentherapie zu verwenden.
- Diagnostik: Verfahren, um eine Krankheit zu identifizieren.
- Wirksamkeitsuntersuchung: Untersuchung mit dem Ziel die Wirksamkeit einer Verbindung nach Einwirkung auf einen lebenden Organismus zu untersuchen.

In einer bevorzugten Ausführungsform des Verfahrens wird die Zelle vor dem Schritt (a) gentechnisch manipuliert. Dabei wird genetisches Material in die Zelle eingebracht, insbesondere eine oder mehrere Polynukleolidsequenzen. In einer weiter bevorzugten Variante dieser Ausführungsform erlaubt die gentechnische Manipulation die Messung mindestens eines der durch die Testsubstanz veränderten funktionellen Parameter. In dieser Ausführungsform werden durch gentechnische Manipulation Voraussetzungen geschaffen unter denen die Veränderung eines funktionellen Parameters überhaupt oder verbessert gemessen werden kann. Dabei ist es insbesondere bevorzugt, daß durch die gentechnische Manipulion eine in der Zelle nicht endogen exprimierte Form eines G-Proteins exprimiert oder ein Reportergen eingeführt wird. Darunter ist insbesondere die gentechnische Einführung eines endogen nicht vorhandenen oder physiologisch nicht exprimierten G-Proteins (GTPbindenden Proteins) in die Zelle zu verstehen, beispielsweise die Einführung eines chimären G-Proteins, das eine Veränderung des Signalweges erlaubt oder eines promiskuitiven G-Proteins, das sehr bindungsfreudig ist. Die Einführung eines Reportergens wiederum erlaubt die Messung einer (extrazellulär ausgelösten) induzierten Expression des Genproduktes.

In einer weiteren bevorzugten Ausführungsform wird die Zelle gentechnisch so manipuliert, daß die Zelle mindestens ein Polynukleotid gemäß einer der Abbildungen 1a), 1c), 1e), 2a), 2c) oder 2e) oder ein dazu zu mindestens 90 % ähnliches Polynukleotid enthält. Damit kann beispielsweise erreicht werden, daß ein Protein, das in der im Verfahren verwendeten Zelle oder Präparation nicht endogen exprimiert wird, von der Zelle synthetisiert wird. Dabei ist es insbesondere bevorzugt, wenn das Polynukleotid in einem rekombinanten DNA-Konstrukt enthalten ist. Unter einem (rekombinanten) DNA-Konstrukt versteht man ein in-vitro hergestelltes DNA-Molekül.

Wenn beim Verfahren vor dem Schritt a) die Zelle gentechnisch manipuliert wird, ist es bevorzugt, daß die Zelle nach der gentechnischen Manipulation und vor dem Schritt (a) unter Bedingungen, die eine Expression erlauben, kultiviert wird, gegebenenfalls unter Selektionsdruck. Unter kultivieren versteht man, Zellen oder Gewebe bei Bedingungen, die ein Überleben der Zellen, bzw. deren Nachfolgegeneration sichern, zu halten. Dabei sollten die Bedingungen hier so gewählt werden, daß eine Expression des durch die gentechnische Manipulation eingefügten Materials ermöglicht wird. Dazu sollten pH, Sauerstoffgehalt, und Temperatur physiologisch gehalten sein und ausreichend Nährstoffe und notwendige Cofaktoren beigefügt sein. Der Selektionsdruck erlaubt, nur die Zellen weiter zu kultivieren, bei denen die gentechnische Manipulation zumindest teilweise erfolgreich war. Dazu gehört beispielsweise die Einführung einer Antibiotikaresistenz über das DNA-Konstrukt.

Es ist bei dem erfindungsgemäßen Verfahren besonders bevorzugt, wenn die verwendete Zelle eine Amphibienzelle, Bakterienzelle, Hefezelle, Insektenzelle oder eine immortalisierte oder native Säugetierzelle ist. Beispiele für Amphibienzellen sind Xenopus Oocyten, für Bakterienzellen E-coli-Zellen, für Hefezellen solche auch Saccharomyces cerevisiae, für Insektenzellen Sf9-Zellen, für immortalisierte Säugetierzelle HeLa-Zellen und für native Säugetierzellen die CHO (Chinese Hamster Ovary)-Zelle.

Bei einer ersten erfindungsgemäß verwendeten Meßmethode zur Feststellung der Bindung der Substanz an ein Protein im erfindungsgemäßen Verfahren erfolgt die Messung der Bindung über die Verdrängung eines bekannten markierten Liganden vom Protein und/oder über die daran gebundene Aktivität einer markierten Testsubstanz. Dabei ist ein Ligand ein mit hoher Spezifität an das Protein bindendes Molekül, das durch eine ebenfalls bindende, zu testende Substanz aus der Bindungsstelle verdrängt wird. Unter Markierung ist eine den Nachweis erleichternde künstliche Modifikation am Molekül zu verstehen. Beispiele sind radioaktive, fluoreszierende oder lumineszierende Markierung.

Bei einer zweiten erfindungsgemäß verwendeten Meßmethode zur Feststellung der durch die Bindung der Substanz an ein Protein im erfindungsgemäßen Verfahren ausgelösten Veränderung der funktionellen Parameter, erfolgt die Messung mindestens eines der durch die Testsubstanz veränderten funktionellen Parameter über Messung der Regulation, Hemmung und/oder Aktivierung von Rezeptoren, Ionenkanälen und/oder Enzymen, insbesondere über Messung der Veränderung der Genexpression, des Ionenmilieus, des pH oder des Membranpotentials, über Veränderung der Enzymaktivität oder der Konzentration der 2^{nd} messenger. Damit ist auf der einen Seite direkt die Messung der Wirkung der Substanz über die Beeinflußung von Rezeptoren, Ionenkanälen und/oder Enzymen erfaßt, auf der anderen Seite als bevorzugt zu messende Beispiele sich ändernder Parameter wie Genexpression, Ionenmilieu, pH, Membranpotential, Enzymaktivität oder Konzentration der 2^{nd} messenger. Dabei versteht man unter Ionenmilieu insbesondere die Konzentration eines oder mehrer Ionen in einem Zellkompartiment, insbesondere dem Cytosol, unter Membranpotential die Ladungsdiffferenz zwischen zwei Seiten einer Biomembran und unter 2^{nd} messenger Botenstoffe des intrazellulären Signalwegs wie z.B. zyklisches AMP (cAMP), Inosotoltriphosphat (IP3) oder Diacylglycerol (DAG).

In einer bevorzugten Variante des Verfahrens wird das Protein in den Schritten (a) und (b) ausgewählt aus:
- der PIM-1-Kinase,
- einem Protein, für das ein Polynukleotid gemäß einer der Abbildungen 1a), 1c) oder 1e) oder ein dazu zu mindestens 90 %, vorzugsweise 95%, insbesondere 97%, ähnliches Polynukleotid kodiert,
- einem Protein mit einer Aminosäuresequenz gemäß einer der Abbildungen 1b), 1d) oder 1f), oder einem dazu zu mindestens 90 %, vorzugsweise 95%, insbesondere 97%, ähnlichem Protein und/oder
- einem Protein, das durch eine Nukleinsäure kodiert wird, die unter stringenten Bedingungen an ein Polynukleotid gemäß einer der Abbildungen 1a), 1c) oder 1e) oder deren Antiisense Polynukleotide bindet.

Darunter erfaßt ist im erfindungsgemäßen Verfahren die Verwendung von Proteinen mit bekannter Sequenz und Funktion, ohne daß für diese im Stand der Technik eine Funktion im Schmerz bekannt war.

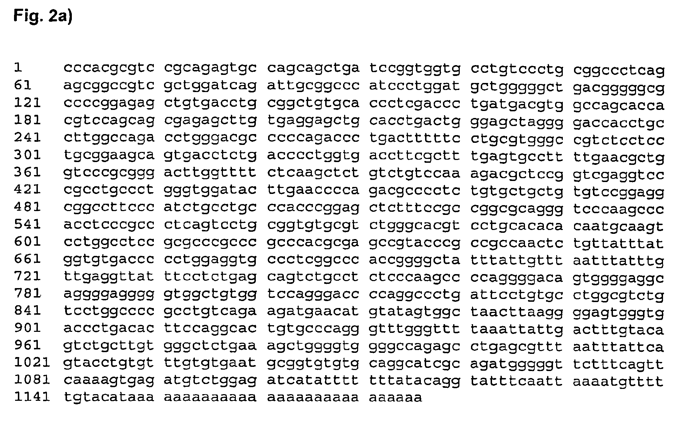

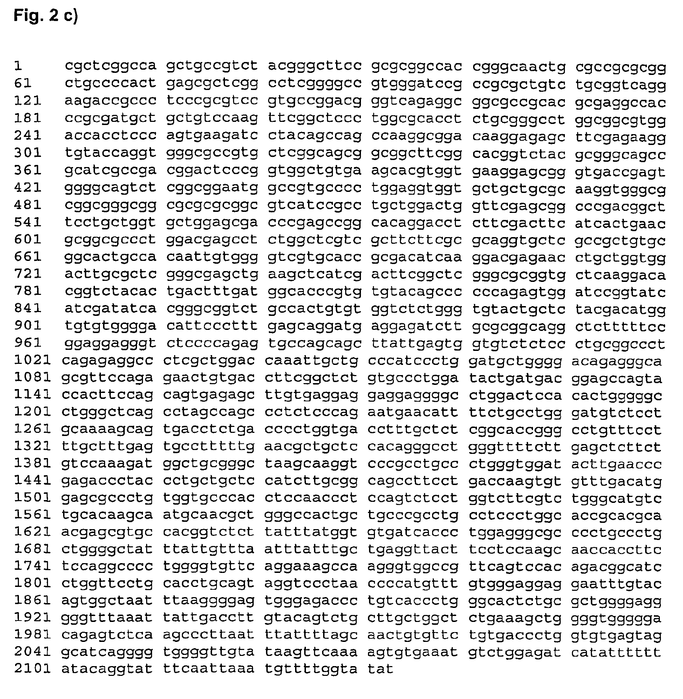

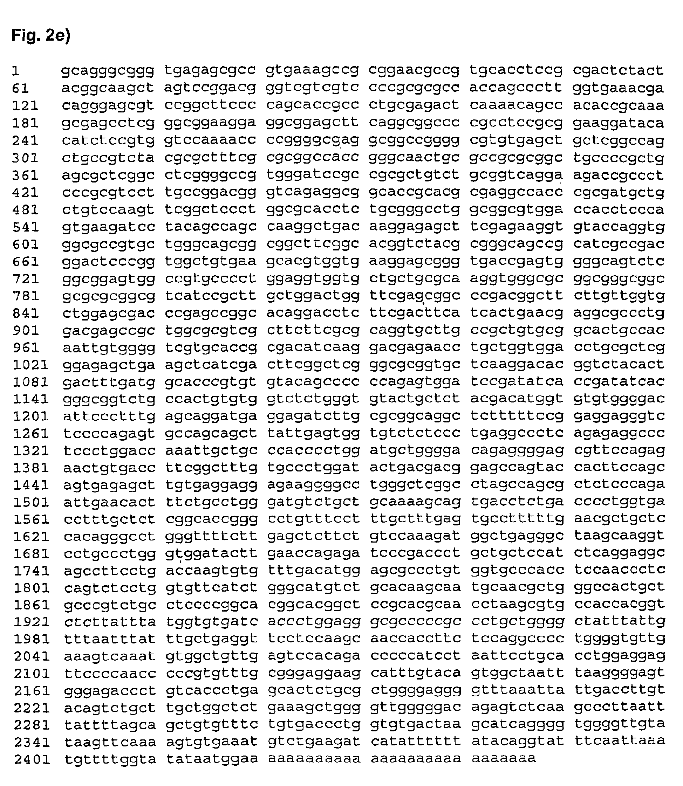

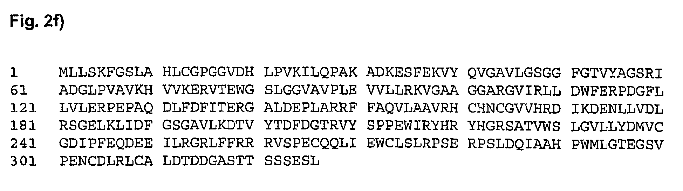

Erfindungsgemäß wird auch ein Polynukleotid verwendet, welches einer der in einer der Abbildungen 2a) und 2e) dargestellten Nukleotidsequenzen zu wenigstens 90%, vorzugsweise 95%, insbesondere zu wenigstens 97% entspricht. Hiervon sind die dargestellten Genfragmente selbst umfaßt, wie auch ein Polynukleotid, das entweder vollständig oder zumindest Teilen der kodierenden Sequenz des dem Fragment entsprechenden Gens entspricht. Damit sind auch Polynukleotide gemeint, die mindestens 90%-ige, vorzugsweise 95%-ige, insbesondere wenigstens 97%-ige Übereinstimmung in der Basenabfolge mit der kodierenden Sequenz der abgebildeten Polynukleotide oder der kodierenden Sequenz der Gens aufweisen.

Es ist weiter bevorzugt, daß es sich bei dem Polynukleotid um RNA bzw. ein- oder doppelstängige DNA, insbesondere mRNA oder cDNA handelt.

Ebenso ist es bevorzugt, daß es sich bei dem Polynukleotid um ein Antisense-Polynukleotid oder PNA handelt, das eine Sequenz aufweist, die in der Lage ist, spezifisch an ein erfindungsgemäßes Polynukleotid zu binden. Dabei versteht man unter PNA "peptidic nucleic acid" (peptidische Nukleinsäure), die zwar die Basenpaare trägt, aber deren Rückrat peptidisch gebunden ist. Ein Antisense-Polynukleotid zeigt die komplementäre Basenabfolge zu mindestens einem Teil einer Basis-Nukleinsäure. Ebenfalls bevorzugt ist es, daß das Polynukleotid Teil eines Ribozym oder sonstigen DNA-Enzyms oder einer katalytischen RNA bzw. DNA ist. Unter Ribozym ist eine katalytisch aktive Ribonukleinsäure zu verstehen, unter DNA-Enzym ein entsprechende Desoxyribonukleinsäure, also katalytische RNA beziehungsweise DNA.

Es ist auch möglich in Sinne dieser Erfindung, wenn das verwendete Protein posttranslational modifiziert wurde, es insbesondere glykosyliert phosphoryliert, amidiert, methyliert, acetyliert, ADP-ribosyliert, hydroxyliert, mit einem Membrananker versehen, gespalten oder verkürzt wurde. Posttranslationale Modifikationen sind beispielsweise dem Voet/Voet. Biochemistry, 1^{st} Edition, 1990, S. 935-938 zu entnehmen.

Eine durch ein erfindungsgemäßes Verfahren dentifizierbare Verbindung ist bevorzugt eine schmerzregulierende Substanz Hierbei bezieht sich Verbindung insbesondere auf niedermolekulare Wirkstoffe, aber auch auf Peptide, Proteine und Nukleinsäuren. Dabei bedeutet identifizierbar, daß die Verbindung das Merkmal aufweist, daß es beim erfindungsgemäßen Screeningverfahren bezüglich der Bindung deutlich stärker, vorzugsweise doppelt so stark bindet wie der Durchschnitt der zu testenden Substanzen oder bezüglich der Änderung der funktionellen Parameter deutlich vom Durchschnitt der zu testenden Substanzen abweicht.

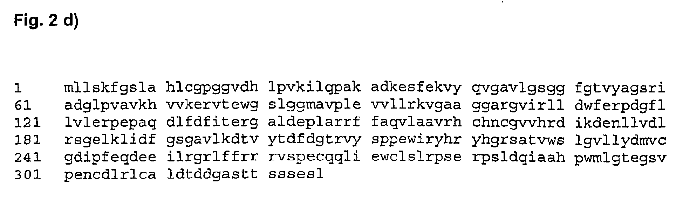

In einer besonders bevorzugten Ausführungsform des erfindungsgemäßen Verfahrens ist in einem Teilverfahren das Protein in den Schritten (a) und (b) ausgewählt aus:
- der PIM-1-Kinase,
- einem Protein, für das ein Polynukleotid gemäß einer der Abbildungen 1a). 1c) oder 1e) oder ein dazu zu mindestens 90 %, vorzugsweise 95%, insbesondere 97%, ähnliches Polynukleotid kodiert,
- einem Protein mit einer Aminosäuresequenz gemäß einer der Abbildungen 1b), 1d), 1f), oder einem dazu zu mindestens 90 %, vorzugsweise 95%, insbesondere 97%, ähnlichem Protein und/oder
- einem Protein, das durch eine Nukleinsäure kodiert wird, die unter stringenten Bedingungen an ein Polynukleotid gemäß einer der Abbildungen 1a), 1c) oder 1e) oder deren Antisense Polynukleotide bindet
und in einem anderen Teilverfahren das Protein in den Schritten (a) und (b) ausgewählt aus:
- der PIM-2-Kinase,
   oder
- der PIM-3-Kinase,
- einem Protein, für das ein Polynukleotid gemäß einer der Abbildungen 2a), 2c) oder 2e) oder ein dazu zu mindestens 90 %, vorzugsweise 95%, insbesondere 97%, ähnliches Polynukleotid kodiert,
- einem Protein mit einer Aminosäuresequenz gemäß einer der Abbildungen 2d), 2f), oder einem dazu zu mindestens 90 %, vorzugsweise 95%, insbesondere 97%, ähnlichem Protein und/oder
- einem Protein, das durch eine Nukleinsäure kodiert wird, die unter stringenten Bedingungen an ein Polynukleotid gemäß einer der Abbildungen 2a), 2c) oder 2e) oder deren Antisense Polynukleotide bindet,
, wobei - meist anschließend - die Ergebnisse aus Schritt b) der Teilverfahren differentiell verglichen werden.

Insgesamt ist eine wichtige Grundlage der Erfindung die Identifizierung schmerzregulierter Gene und Genfragmente. Darauf basiert das Screeningverfahren. Im folgenden werden weitere Ausführungsbeispiele erläutert.

### 1. Therapie chronischer Schmerzen

Die mRNA-Expression der Kinasen wurde durch in-situ-Hybridisierung im Rückenmarksgewebe untersucht. Im Rückenmark projizieren die primären sensorischen Neurone auf nachgeschaltete zentralnervöse Neurone, es handelt sich hierbei neben supraspinalen Vorgängen um die zentrale Umschaltstelle für nozizeptive Information. Zahlreiche Experimente konnten zeigen, daß der Entwicklung chronischer Schmerzzustände plastische Veränderungen des Nervensystems zugrundeliegen (als Überblick siehe Corderre et al., 1993 ; Zimmermann und Herdegen, 1996). Insbesondere in den Neuronen der dorsalen Wurzelganglien und des Rückenmarks sind plastische Veränderungen beschrieben worden, die mit der Regulation schmerzrelevanter Gene einhergeht. So ist für eine Reihe von Neurotransmitter-Rezeptoren, die für die Schmerztherapie von Bedeutung sind, eine Gen-Regulation im Rückenmark beschrieben worden (siehe Tabelle 1). Auf dieser Grundlage könnten die gefundenen, unter Schmerz regulierten cDNA-Sequenzen zur Therapie (Gentherapie, Antisense, Ribozyme) und Diagnose chronischer Schmerzzustände verwendet werden.

### 1.1 Antisense-Strateglen

Hierbei werden, abgeleitet von der Nukleinsäuresequenz der vollständigen cDNA oder von Teilbereichen Konstrukte erstellt, die die mRNA oder Proteinkonzentration herabsetzen können. Dies können z.B. antisense-Oligonukleotide (DNA oder RNA) sein, die eventuell unter Verwendung modifizierter Nukleotidbausteine (z.B. O-Allyl-Ribose) eine erhöhte Stabilität gegenüber Nukleasen aufweisen. Zudem ist die Verwendung von Ribozymen denkbar, die als enzymatisch aktive RNA-Moleküle eine spezifische Spaltung der RNA katalysieren. Daneben könnten auch Vektoren eingesetzt werden, die die erfindungsgemäß verwendeten Sequenzen dieser Nukleotidsequenzen unter Kontrolle eines geeigneten Promotors exprimieren und somit für eine in-vivo oder ex-vivo Therapie geeignet sind. Zusätzlich sind auch Antisense-Konstrukte möglich, die unter Austausch des Phosphatrückgrats von Nukleotidsequenzen (z.B. PNAs, d.h. Peptide Nucleic Acids) oder Verwendung nichttraditioneller Basen wie inosin, Queosin oder Wybutosin sowohl wie Acetyl-, Methyl-, Thio- und ähnlich modifizierte Formen von Adenin, Cytidin, Guanosin u, Thymidin und Uridin nicht oder in geringerem Masse durch endogene Nukleasen abgebaut werden können.

### 1.2. Antagonisten/ Agonisten bzw. Inhibitoren/Aktivatoren der im Screeningverfahren erfindungsgemäß verwendeten Genprodukte.

Dies umfaßt Substanzen, die durch eine Bindung an das Genprodukt dessen Funktion verändern. Dies können sein:
1.2.1. Organisch-chemische Moleküle, die im Rahmen eines Wirkstoffscreenings unter Verwendung der Genprodukte der erfindungsgemäß verwendeten cDNA als Bindungspartner gefunden werden.
1.2.2. Antikörper, seien es polyklonale, chimäre, single-chain, F_{ab}-Fragmente oder Fragmente aus Phagen-Banken, die bevorzugt als neutralisierende Antikörper über eine Bindung an die Genprodukte spezifisch die Funktion beeinflussen.
1.2.3. Aptamere, d.h. Nukleinsäuren oder Nukleinsäurederivate mit Proteinbindenden Eigenschaften. Dazu gehören auch sog. Spiegelmere, die durch Spiegelevolution gewonnene spiegelbildliche und damit stabile Oligonukleotide darstellen, die hochaffin und hochspezifisch ein Zielmolekül binden können (Klußmann et al., 1996).

### 1.3. Gentherapie

Die beschriebenen Sequenzen können zur Therapie neurologischer Erkrankungen insbesondere chronischer Schmerzustände eingesetzt werden, indem sie nach Klonierung in geeignete Vektoren (z. B. Adenovirus-Vektoren oder adeno-assozierter-Virus-Vektoren) zur in vivo oder ex-vivo Therapie verwendet werden, um dort z.B. einer Überexpression oder Unterexpression des endogenen Genproduktes entgegenzusteuem, die Sequenz des defekten Genproduktes zu korrigieren (z. B. durch Transsplicing mit dem exogenen Konstrukt) oder ein funktionelles Genprodukt zur Verfügung zu stellen.

### 2. Diagnose

Polynukleotidsequenzen (Oligonukleotide, antisense -DNA & RNA-Moleküle, PNAs), die von den im Screeningverfahren etc. verwendeten Nukleotidsequenzen abgeleitet sind, könnten zur Diagnose von Zuständen oder Erkrankungen eingesetzt werden, die mit einer Expression dieser Gensequenzen assoziiert sind. Beispiele dieser Zustände oder Erkrankungen beinhalten neurologische Erkrankungen inklusive chronischer Schmerzen oder neuropathischer Schmerzen (hervorgerufen z.B. durch Diabetes, Krebs oder AIDS) oder neurodegenerativer Erkrankungen wie Alzheimer, Parkinson, Chorea Huntington, Jacob-Creutzfeld, amyotrophe Lateralsklerose und Demenzen. Die Nukleotidsequenzen könne auf vielfältige Weise (Northernblot, Southemblot, FISH-Analyse, PRINS-Analyse, PCR) entweder zur Identifizierung der Genproduktes oder abweichender diagnostisch relevanter Genprodukte oder zur Quantifizierung des Genproduktes dienen. Neben der Nukleinsäurediagnostik können auch Antikörper oder Aptamere gegen das von den erfindungsgemäßen verwendeten Nukleinsäuren kodierte Protein zur Diagnostik eingesetzt werden (z. B. mittels ELISA, RIA, immuncytochemische oder immunhistochemische Verfahren), um das Protein oder abweichende Formen zu identifizieren und das Protein zu quantifizieren.

Im Hinblick auf eine Gendiagnostik könnten Nukleinsäure-Sonden abgeleitet von den erfindungsgemäß verwendeten Nukleotidsequenzen zur Bestimmung des Gen-Lokus eingesetzt werden (z.B. durch FISH, FACS, artifizielle Chromosomen wie YACs, BACs oder P1-Konstrukte).

Die folgenden Beispiele und Abbildungen sollen die Erfindung erläutern, ohne sie darauf zu beschränken.

### Abbildungen und Beispiele

### Abbildungen:

Fig. 1a) cDNA-Sequenz von PIM1-Kinase, human; AN: NM_002648
Fig. 1b) Aminosäure-Sequenz von PIM1-Kinase, human; AN: NP_002639
Fig. 1c) cDNA-Sequenz von PIM1-Kinase, Ratte ; AN: NM_017034
Fig. 1d) Aminosäure-Sequenz von PIM1-Kinase, Ratte; AN: NP_058730
Fig. 1e) cDNA-Sequenz von PIM1-Kinase, Maus; AN: NM_008842
Fig. 1f) Aminosäure-Sequenz von PIM1-Kinase, Maus; AN: NP_032868
Fig. 2a) mRNA-Sequenz ähnlich zu PIM3-Kinase, human; AN: BC017083
Fig. 2c) cDNA-Sequenz von PIM3-Kinase, Ratte; AN: NM_022602
Fig. 2d) Aminosäure-Sequenz von PIM3-Kinase, Ratte; AN: NM_072124
Fig. 2e) cDNA-Sequenz von PIM3-Kinase, Maus; AN: BC017621
Fig. 2f) Aminosäure-Sequenz von PIM3-Kinase, Maus; AN: BC017621
Fig. 3) mRNA-Expression der PIM-Kinasen im Lumbalmark der adulten Ratte. s. Beispiel 1a)
Fig. 4) Veränderungen der PIM-1 Genexpression im Rückenmark (L5) nach Ischiadicus-Ligatur (Bennett); s. Beispiel 1 b)
Fig. 5) PIM-1 mRNA-Spiegel im Lumbalmark (L5) nach Bennett-Ligatur Quantitative Auswertung der in situ-Hybridisierungsergebnisse
Fig. 6) mRNA-Expressionsmuster der PIM-Kinasen im Hinterhom des Rückenmarks.
Fig. 7) mRNA-Expression der PIM-Kinasen im Spinalganglion
Fig. 8) Lokalisation der PIM-1 Genexpression im Spinalganglion (L6) der Ratte mit *in situ*-Hybridisierung
Fig. 9) Veränderungen der PIM-1, PIM-2 und PIM-3 mRNA-Spiegel im Spinalganglion L6 nach bilateraler CFA-Arthritis Densitometrische Analyse der Ethidiumbromid-gefärbten Banden für PIM-1, PIM-2, PIM-3 und GAPDH nach elektrophoretischer Auftrennung der PCR-Produkte (25 Zyklen). Darstellung der Meßwerte als Quotient aus PIM-1 und GAPDH bzw. PIM-2/GAPDH bzw. PIM-3/GAPDH

### Beispiele:

### Beispiel 1

### Identifizierung schmerzregulierter Gene

Es wurde folgendes Vorgehen gewählt:
Als Ausgangspunkt für die Isolierung schmerzregulierter Gene wurde das sogenannte Formalinmodell an der Ratte gewählt, bei dem Formalin in die Rattenpfote injiziert wird. Das Zielgewebe, in dem die schmerzregulierte Expression der erfindungsgemäß verwendeten Gene nachgewiesen wurde, war der dorsale Teil des Rückenmarks der Ratte in den Segmenten L3-L6.
**Tiermodell :** Der Formalin-Test stellt ein geeignetes Modell für den Bereich des inflammatorischen/persistenten Schmerz dar (Duibisson et al., 1997). Hierbei wurde 50µl 5%ige Formalinlösung uniliateral in die Hinterpfote adulter Wistar-Ratten injiziert und die Tiere 24Std. nach der Injektion zur Gewebeentnahme getötet. Parallel wurden bei den Kontrolltieren isotonische Kochsalzlösung in die Hinterpfote injiziert.
**Gewebeentnahme**. Die Tier werden dekapitiert, die Rückenwirbelsäule herauspräpariert, Schnitte des Rückenmarks angefertigt und mit spezifischen markierten Antikörpern gegen die PIM-Kinasen hybridisiert.

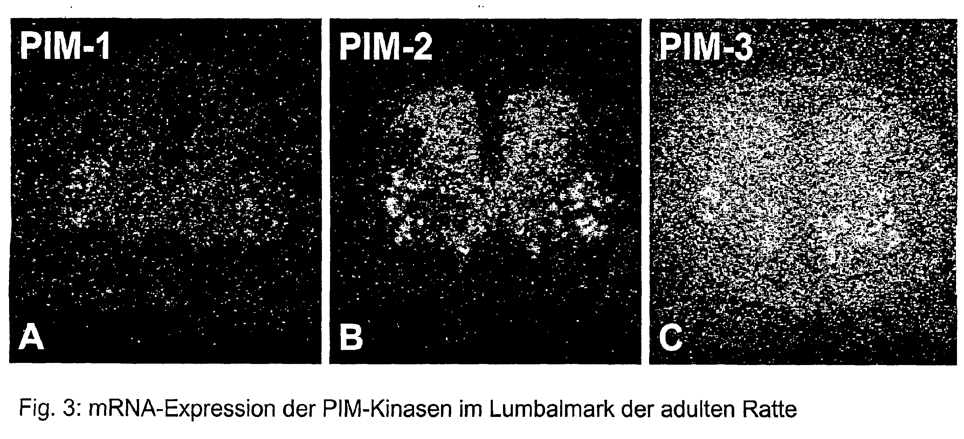

### Beispiel 1a) zu Fig. 3)

Digitalisierte Röntgenfilmautoradiogramme von Gefrierschnitten durch das Rückenmark (Ebene L5) nach *in situ*-Hybridisierung mit spezifischen ³⁵S-markierten RNA-Sonden. Die Expositionszeit der Röntgenfilme betrug 72h. PIM-1 ist unter normalen Bedingungen relativ gering exprimiert (A). Im Vergleich dazu ist PIM-2 konstitutiv stark exprimiert (B) mit deutlicher Dominanz in der grauen Substanz (vor allem im oberflächlichen Hinterhom und in den Motoneuronen des Vorderhoms). PIM-3 mRNA ist über den ganzen Schnitt verteilt (C), mit stärkeren Signalen über den neuronalen Bereichen und schwachen Signalen über der weißen Substanz.

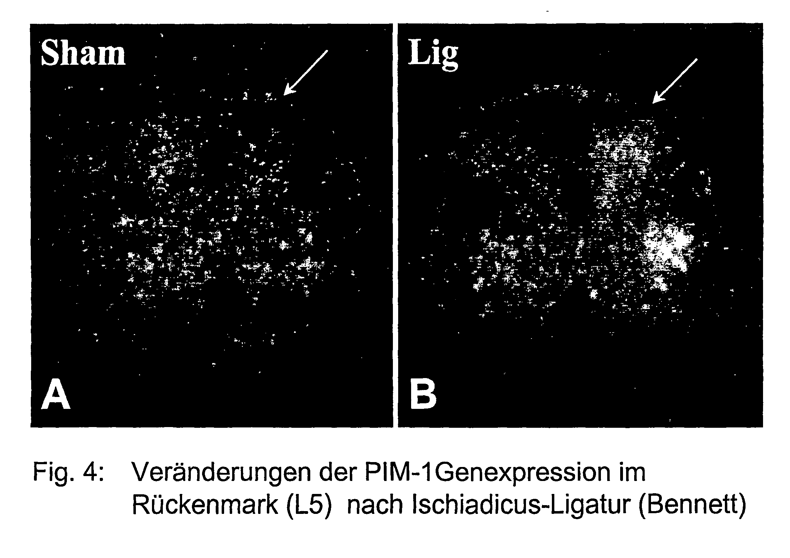

### Beispiel 1b) zu Fig. 4

Vestärkte PIM-1 Genexpression im Rückenmark nach Ischiadicus-Ligatur. Digitalisierte Gefrierschnitte durch das Lumbalmark von Tieren 7 Tage nach Bennett-Ligatur (B) zeigen nach Hybridisierung mit einer radioaktiv markierten PIM-1 Sonde eine deutliche Erhöhung der PIM-1 mRNA-Spiegel Rückenmarkshälfte ipsilateral zur Ligation (Pfeil), sowohl im Hinterhorn als auch im Vorderhom. In Sham-operierten Tieren (A) wird diese Erhöhung nicht beobachtet

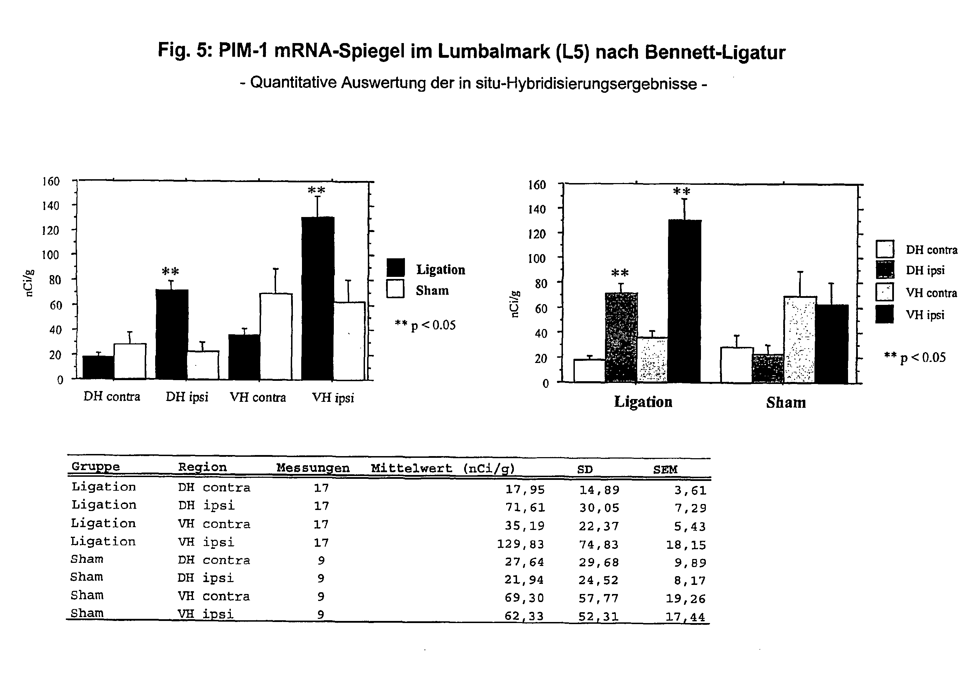

### Beispiel 1c) zu Fig. 5

Semi-quantitative Analyse der PIM-1 mRNA Spiegel in den verschiedenen Rückenmarksregionen von Sham-operierten Tieren und von Tieren 7 Tage nach Bennett-Ligatur.
Nach Digitalisierung der mit einer PIM-1 spezifischen Sonde hybridisierten Gefrierschnitte (MCID Bildanalyse-System, Imaging Research, Canada) werden die radioaktiven Hybridisierungssignale densitometrisch erfaßt. Die Meßwerte werden nach Etablierung einer Standardkurve in nCi/g Gewebe umgewandelt.
Für die Etablierung einer Standardkurve werden Objektträger mit ¹⁴C-Plastikstreifen mit definiertem radioaktiven Gehalt (American Radiolabeled Chemical Inc.) gemeinsam mit den hybridisierten Schnitten auf Röntgenfilm für die gleiche Dauer exponiert.
Analysiert wurden jeweils die kontra- und ipsilaterale Regionen des Hinterhoms sowie des Vorderhoms.
Für die Gruppe der Sham-operierten Tiere wurden pro Region 9 Messungen, für die Gruppe nach Bennett-Ligatur 17 Messungen durchgeführt.

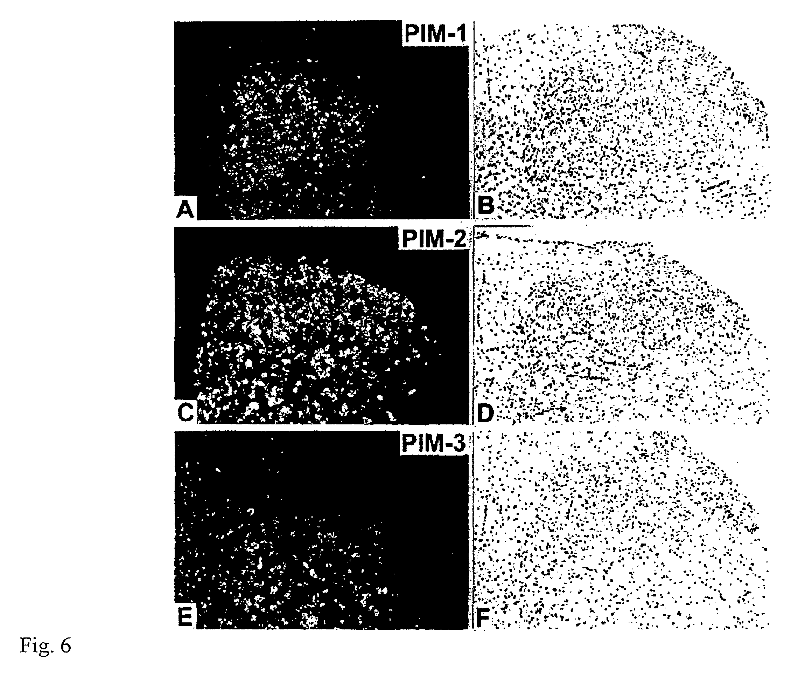

### Beispiel 1d)zu Fig. 6

Hochauflösende Dunkelfeldaufnahmen des oberflächlichen Hinterhorns ipsilateral zur Ischiadicus-Ligatur nach Hybridisierung mit PIM-spezifischen Sonden (A,C, E) und Gegenfärbung mit Kresylviolett (B,D,F). PIM-1 hybridisierte Schnitte zeigen eine "layer"-spezifische Expression von PIM-1 in Lamina 2 und 3 (A,B). PIM-2 Transkripte sind besonders in Lamina 1 und Lamina und im gesamten Hinterhorn relativ stark exprimiert (D,E).

Hingegen ist PIM-3 weniger im oberflächlichen Hinterhorn, sondern in Lamina 3 und den tieferen Schichten exprimiert (E,F).

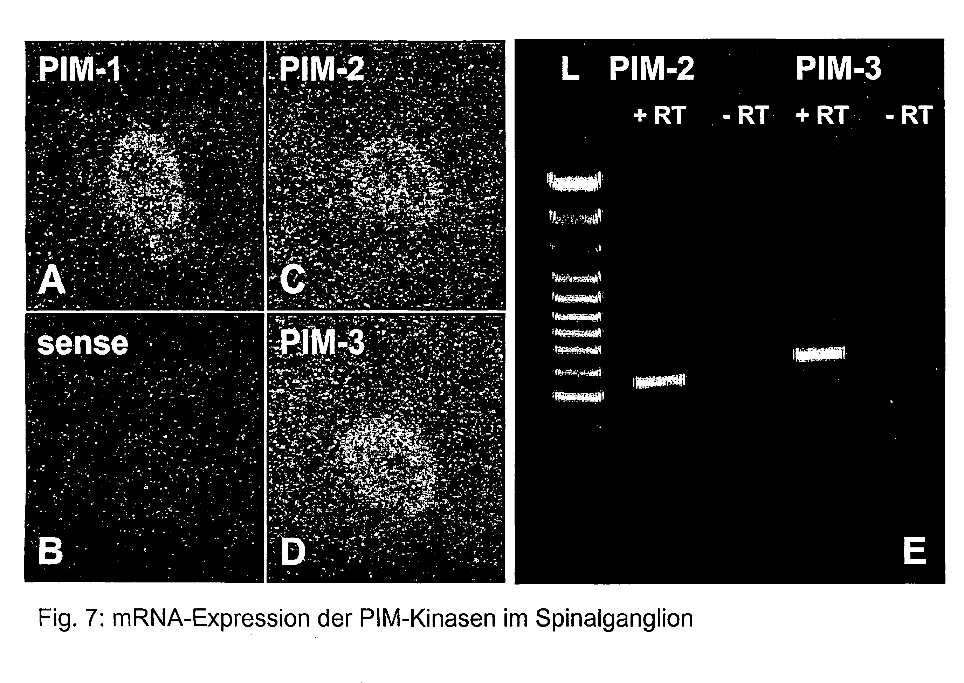

### Beispiel 1e) zu Fig. 7

Digitalisierte Röntgenfilmautoradiogramme von Gefrierschnitten durch ein lumbales Spinalganglion (L6) nach *in situ*-Hybridisierung mit spezifischen ³⁵S-markierten RNA-Sonden. Die Expositionszeit der Röntgenfilme betrug 72h.
Alle drei PIM-Kinasen sind unter normalen Bedingungen im Spinalganglion exprimiert (A,C,D). Sonden in sense-Orientierung, hier am Beispiel für PIM-1 gezeigt (B), produzieren keine spezifischen Hybridisierungssignale. PIM-3 ist im Vergleich stärker expriomiert (D).
Für die RT-PCR- Analyse wurden die Spinalganglien (L6) aus 5 Kontrolltieren beiderseits entnommen und für die Extraktion der Gesamt-RNA gepoolt. Für die RNA-Extraktion wurde Trizol (Gibco-BRL) verwendet. Vor Durchführung der reversen Transkription wurde eine Dnase I-Behandlung durchgeführt.
Für die reverse Transkription mit Supersript II (Gibco-BRL) wurden 2,5 µg der isolierten Gesamt-RNA in einer 50 µl-Reaktion eingesetzt.
Die PCR-Amplifikation wurde in einem Thermocycler 9700 (Perkin Eimer) nach folgendem Programm durchgeführt:
1 Zyklus 95°C, 3 min; 40 Zyklen (94°C, 45 sec; 60°C, 45 sec; 72°C, 60 sec); 1 Zyklus 72°C, 7 min.
Als Matrize wurde jeweils 7,5 µl der cDNA eingesetzt (50 ng/µl).
Die spezifischen Amplicons für PIM-2 (518 bp) und PIM-3 (612 bp) hatten die erwarteten Größen; GAPDH. Als Negativ-Kontrollen wurden RT-Reaktionen unter Weglassen der reversen Transkriptase durchgeführt.

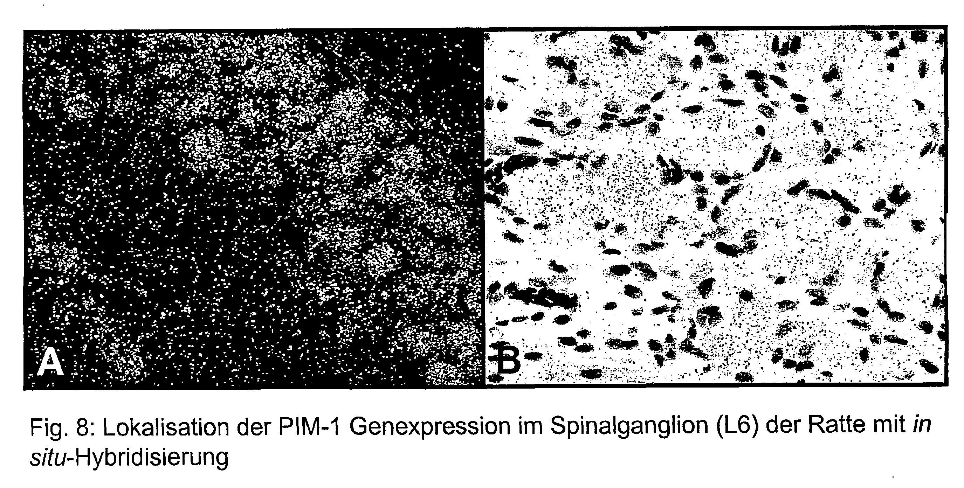

### Beispiel 1f) zu Fig. 8

Hybridisierung von Gefrierschnitten mit PIM-1 spezifischen Sonden zeigt spezifische Hybridisierungssignale über den zellreichen Regionen des Spinalganglions (Dunkelfeld aufnahme in A). Die mikroskopische Hochauflösung im Hellfeld bestätigt, daß die Signale in den Kresylviolett gegengefärbten Schnitten primär über den neuronalen Zellen lokalisiert sind (B).

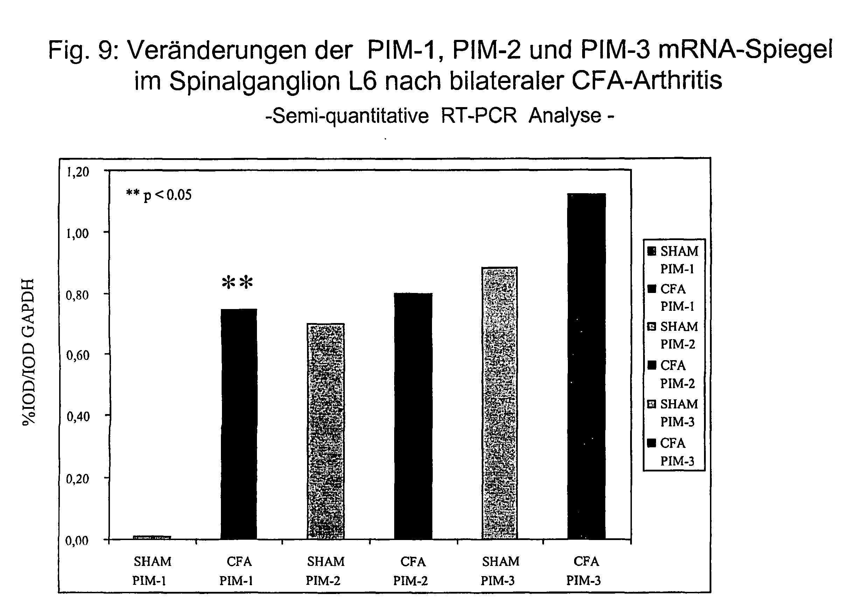

### Beispiel 1g) zu Fig. 9

Aus 5 Tieren wurden beiderseits die Spinalganglien (L6) entnommen und für die Extraktion der Gesamt-RNA gepoolt.
Für die RNA-Extraktion wurde Trizol (Gibco-BRL) verwendet. Vor Durchführung der reversen Transkription wurde eine Dnase 1-Behandlung durchgeführt.
Für die reverse Transkription mit Supersript II (Gibco-BRL) wurden 2,5 µg der isolierten Gesamt-RNA in einer 50 µl-Reaktion eingesetzt.
Die PCR-Amplifikation wurde in einem Thermocycler 9700 (Perkin Elmer) nach folgendem Programm durchgeführt:
1 cycle 95°C, 3 min ; 40 cycles (94°C, 45 sec; 60°C, 45 sec; 72°C, 60 sec) ; 1 cycle 72°C, 7 min.
Als Matrize wurde jeweils 7,5 µl der cDNA eingesetzt (50 ng/µl).
Nach 10, 15, 20, 25, 30, 35 und 40 Zyklen wurden jeweils 10 µl der PCR-Reaktion entnommen und gelelektrophoretisch aufgetrennt, um den linearen Bereich der Amplifikation zu bestimmen. Die Ethidiumbromid-gefärbten Gele wurden digitalisiert und die PCR-Banden densitometrisch gemessen.
Die spezifischen Amplicons hatten die erwarteten Größen (PIM-1, 550 bp; PIM-2, 518 bp; PIM-3, 612 bp; GAPDH, 227 bp).
Um die Veränderung der PIM-Expression nach CFA zu erfassen, wurden die Quotienten aus den spezifischen Amplicons (PIM-1, PIM-2, PIM-3) und dem nicht regulierten GAPDH für jede Versuchsgruppe gebildet.

### Umfassende Expressionsanalyse aller drei Mitglieder der Pim-Kinase-Familie: PIM1, PIM2 und PIM3

Die umfassende Expressionsanalyse aller drei Mitglieder der Pim-Kinase-Familie: PIM1, P1M2 und PIM3 ; wird im Folgenden tabellarisch dargestellt.

| | **P1M-1** | | **PIM-2** | | **PIM-3** | |
|---|---|---|---|---|---|---|
| **Expression** | **Neurone** | **Gliazellen** | **Neurone** | **Gliazellen** | **Neurone** | **Gliazellen** |
| Rückenmark | ++ | + | +++ | + | + | +++ |
| DRG | ++ | + | +++ | + | + | +++ |

Die ISH-Ergebnisse zeigen folgende Expressionsmuster für die PIM-Kinasen:
• Eine neuronale Lokalisation von PIM-1, PIM-2 und PIM-3 mRNA im Rückenmark (ISH, RT-PCR)
• Eine neuronale Expression von PIM-1, PIM-2 und PIM-3 mRNA im DRG der Ratte. PIM-2 und PIM-3 scheinen auch in nicht-neuronalen Zellen vorzukommen. Die immunhistochemischen Daten zeigen das PIM-1 Protein in DRG-Neuronen und das PIM-2 Protein in DRG-Neuronen sowie in Gliazellen.

Für PIM-1 war eine erhöhte mRNA Expression in mehreren Schmerzmodellen nachweisbar:
• Hochregulation der PIM-1 mRNA in DRG-Extrakten im CFA-Modell
• Hochregulation von PIM-1 mRNA im Dorsalhorn nach lschiadicus-Ligatur der Ratte. Daneben findet man auch in Motomeuron-Arealen des Vorderhoms eine Hochregulierung.
• PIM1 im neuropathischem Schmerz Hochregulation in Mikroglia (C1q) und in Neuronen
• Im Gegensatz zu PIM-1 ist PIM-2 schon konstitutiv im Hinterhom recht stark exprimiert, wobei die im Bennett-Modell beobachtete geringe Hochregulation nach statistischer Auswertung nicht signifikant war.
• Zunahme der neuronalen PIM-1 Immunreaktivität im Hinterhom im Chung-Modell.
• Die PIM-3 mRNA wird im tieferen Hinterhom als auch im Vorderhom sowohl neuronal als auch gliär exprimert, nach Läsion aber nicht reguliert.

### Beispiel 2:

### Durchführung des Screeningverfahrens mit Messung der Bindung über die Verdrängung eines radioaktiv markierten Liganden

Ein Nukleinsäureabschnitt, der für die PIM1-Kinase kodiert, wird in einem Expressionsvektor kloniert, der eine konstitutive Expression (z.B. CMV-Promoter) oder eine induzierbare Expression in eukaryoten Zellen erlaubt. Die DNA wird mit geeignetem Transfektionsverfahren, z.B. mit Lipofectamin (Fa. Roche Diagnostics) in eukaryotischen Zellen (z.B. CHO-Zellen, HEK293-Zellen oder NIH-3T3-Zellen) hineingebracht. Die Zellen werden in Anwesenheit eines Selektionsreagenzen (z.B. Zeocin, Hygromycin oder Neomycin) kultiviert so daß nur die Zellen überleben, die das DNA-Konstrukt aufgenommen haben, und bei länger andauernder Selektion, auch in das Genom inkorporiert haben.
Ausgehend von diesen Zellen werden Membranfraktionen gewonnen, die die PIM1-Kinase in großer Menge enthalten und für einen Bindungsassay verwendet werden können. Dieser besteht aus 1.) dem PIM1-Kinase enthaltenden Membranen 2.) einem radioaktiv markierten Liganden 3.) einem Bindungspuffer (z.B. 50 mM HEPES pH 7.4, 1 mM EDTA) und dem auf Bindung zu untersuchenden Liganden. Nach Inkubation der oben genannten Reaktionsgemische (für z.B. 30-60 min) bei einer geeigneten Temperatur (meistens Raumtemperatur) werden die nichtgebundenen radioaktiven Ligandenmoleküle abfiltriert. Die Restmenge an gebundenem Liganden wird nach Zugabe eines Szintillationscocktails in einem β-Counter (z.B. Trilux, Fa. Wallac) vermessen. Zeigt die Testsubstanz eine Bindung an die PIM1-Kinase, so wird dies als verringerter radioaktiver Einbau detektiert. Dieses Verfahren wird geeignetermaßen so miniaturisiert, daß es in (96-, 384- oder 1536-well) Mikrotiterplatten durchgeführt werden kann, um dieses Verfahren mittels eines Roboters im sogenannten Hightroughput-Screening (HTS)-Verfahren durchzuführen.

### Beispiel 3:

### Durchführung des erfindungsgemäßen Screeningverfahrens mit Messung der durch die Bindung der Substanz veränderten funktionellen Parameter

Ein Nukleinsäureabschnitt, der für die Proteinkinase PIM-1 kodiert, wird in einem Expressionsvektor kloniert, der eine induzierbare Expression in Prokaryonten, wie z.B. E.coli erlaubt. Hierbei wird der Nukleinsäureabschnitt so modifiziert, daß er als Fusionsprotein mit einer zusätzlichen N- oder C-terminalen Aminosäuresequenz exprimiert wird. Diese Sequenz sollte bei unveränderter Funktion der PIM-1 Kinase eine Aufreinigung über ein spezifisches Verfahren erlauben, z.B. Glutathion S-Transferasefragment, das über Bindung an Glutathion eine Isolierung aus dem Proteingemisch erlaubt. Nach Transfektion der Bakterien, Induktion des Genes (z.B. mit IPTG beim lac-Promoter) und Aufschließen der Bakterien werden die Fusionsproteine aufgereinigt und in einem in vitro-Kinase Experiment eingesetzt. Hierbei werden 5 µg Protein bei 30°C für 30 Minuten in 50 µl Kinasepuffer (20 mM PIPES, pH 7.0, 5 mM MnCl₂, 7 mM β-Mercaptoethanol, 0.4 mM Spermin, 10 mM rATP) ergänzt mit 10 µCi [γ³²P] ATP. Als Substrate werden gereinigtes Histon H1-Protein (Fa. Sigma) oder bakteriell exprimiertes GST-NFATc1-Fusionsprotein hinzugegeben. Nach der Inkubationszeit wird das nicht-inkorporierte [γ-³²P] ATP abfiltriert und die Menge an eingebautem ³²Phosphat durch β-Szintillation (Trilux, Fa. Wallac) bestimmt. In einem Experiment zum Aufspüren neuer PIM-1 Kinase-Inhibitoren werden in diesem Ansatz die Testsubstanzen mitinkubiert und eine Abnahme der ³²P-Inkorporation als Indikator für einen Inhibitor benutzt. Dieses Verfahren wird geeignetermaßen so miniaturisiert, daß es in (96-, 384- oder 1536-well) Mikrotiterplatten durchgeführt werden kann, um dieses Verfahren mittels eines Roboters im sogenannten Hightroughput-Screening (HTS)-Verfahren durchzuführen.

### Beispiel 4:

### Beispiel für ein Arzneimittel enthaltend eine Verbindung - Tablettenformulierung

Tabletten können durch direktes Verpressen von Mischungen der nach einen erfindungsgemäßen Verfahren identifizierten Verbindung mit entprechenden Hilfsstoffen oder durch Verpressen von verbindungshaltigen Granulaten (mit gegebenenfalls weiteren Hilfsstoffen) hergestellt werden. Die Granulate können dabei entweder durch Feuchtgranulation mit z.B. wäßrigen Granulierflüssigkeiten und anschließender Trocknung dieser Granulate oder durch Trockengranulation z.B. über Kompaktierung hergestellt werden.

| ■ Direktverpressung | | |
|---|---|---|
| z.B. pro Tablette: | 25 mg | einer nach einem erfindungsgemäßen Verfahren identifizierten Verbindung |
| | 271 mg | LudipressTM (Granulat zur Direkttablettierung aus Lactose monohydrat, Povidon K30 und Crospovidon) |
| | 4 mg | Magnesiumstearat |
| | 300 mg | Gesamt |

Homogene Mischung des Wirkstoffes mit den Hilfsstoffen herstellen und diese auf einer Tablettenpresse zu Tabletten mit einem Ø von 10 mm verpressen.

| ■ Trockengranulation | | |
|---|---|---|
| z.B. pro Tablette: | 25 mg | einer nach einem erfindungsgemäßen Verfahren identifizierten Verbindung |
| | 166 mg | Microcristalline Cellulose |
| | 80 mg | Niedrig substituierte |
| | | Hydroxypropylcellulose (I-HPC LH |
| | | 11^{™}) |
| | 5 mg | Hochdisperses Siliziumdioxid |
| | 4 mg | Magnesiumstearat |
| | 280 mg | Gesamt |

Homogene Mischung der Verbindung mit der Mikrokristallinen Cellulose und der I-HPC herstellen und diese Kopaktieren. Nach dem Sieben der Komprimate wird das entstandene Granulat mit Magnesiumstearat und Siliziumdioxid gemischt und auf einer Tablettenpresse zu Tabletten mit einem Ø von 9 mm verpreßt.

| ■ Feuchtgranulation | | |
|---|---|---|
| z.B. pro Tablette: | 25 mg | einer nach einem erfindungsgemäßen Verfahren identifizierten Verbindung |
| | 205 mg | Mikrokristalline Cellulose |
| | 6 mg | Povidon K30 |
| | 10 mg | Crospovidon |
| | 4 mg | Magnesiumstearat |
| | 250 mg | Gesamt |

Homogene Mischung der Verbindung mit der Mikrokristallinen Cellulose und dem Crospovidon herstellen und diese in einem Granulator mit einer wäßrigen Lösung des Povidons granulieren. Das feuchte Granulat wird anschließend nachgranuliert und nach der Trocknung im Trockenschrank (50°C) 10 h getrocknet. Das trockene Granulat wird mit dem Magnesiumstearat zusammen gesiebt, endgemischt und auf einer Tablettenpresse zu Tabletten mit einem Ø von 8 mm verpreßt.

### Beispiel 5:

### Beispiel für ein Arzneimittel enthaltend nach einem eine erfindungsgemäßen Verfahren identifizierte, Verbindung - parenterale Lösung

1 g einer nach einem erfindungsgemäßen Verfahren identifizierten Verbindung wird in 1 l Wasser für Injektionszwecke bei Raumtemperatur gelöst und anschließend durch Zugabe von NaCl (Natriumchlorid) auf isotone Bedingungen eingestellt.

### Literatur:

Akopian AN, Sivilotti L & Wood JN (1995) Nature 379: 257-262
Ausubel FM, Brent R, Kingdton RE, Moore DD, Seidman JG, Smith JA & Struhl K eds.(1190) Current protocols in molecular biology. John Wiley &Sons, Inc. New York, NY.
Baba H, Doubell TP, Woolf CJ 1999: Peripheral inflammation facilitates Aβ fiber-mediated synaptic input to the substantia gelatinosa of the adult rat spinal cord. J Neurosci 19: 859-867
Bauer D, Müller H, Reich J, Riedel H, Ahrenkiel V, Warthoe P & Strauss M (1993): Identification of differentially expressed mRNA species by an improved display technique (DDRT-PCR) Nucl Acids Res 21: 4272-4280.
Bonini A, Anderson SM, Steiner DF (1997) Molecular cloning and tissue expression of a novel orphan G Protein-coupled receptor from rat lung. Biochem Biophys Res Comm 234: 190-193.
Chih-Cheng et al., (1995): A P2X prinoceptor expressed by a subset of sensory neurons. Nature 377:428-432
Corderre TJ, Katz J, Vaccarino AL, Melzack R (1993): Contribution of central plasticity to pathological pain: review of clinical and experimental evidence. Pain 52: 259-285.
Dickenson (1995) Novel pharmacological targets in the treatment of pain. Pain Rev., 2, 1-12.
Dubuisson et al., 1997 Pain, 4:161-174.
Feng Y & Gregor P (1997) Cloning of a novel member of the G Protein-coupled receptor family related to peptide receptors. Biochem Biophys Res Comm 231: 651-654.
Furukawa T, Yang Y, Nakamoto B, Stamatoyannopoulos G, Papayannopoulou T (1996): Identification of new genes expressed in a human erythroleukemia cell line. Bloods Cell Mol & Dis 22:11-22.
Gunasekar PG, Kanthasamy, AG, Borowitz JL, Isom GE 1995: NMDA receptor activation produces concurrent generation of nitric oxide and reactive oxygen species: implication for cell death. J Neurochem 65: 2016-2021.
Hawes BE, Fried S, Yao X, Weig B, Graziano MP 1998: Nociceptin (ORL1) and µ-Opioid receptors mediate mitogen-activated protein kinase activation in CHO cells through a Gi-coupled signaling pathway: evidence for distinct mechanisms of agonist-mediated desensitization. J Neurochem 71: 1024-1033.
Hubank M & Schatz DG (1994): Identifying differences in mRNA expression by representational difference analysis of cDNA. Nucl Acids Res 22: 5640-5648.
Klußmann S et al., 1996: Nature Biotechnology 14: 1112-1115.
Li L-Y & Chang K-J 1996: The stimulatory effect of opioids on mitogen-activated protein kinase in chinese hamster ovary cells transfected to express µ-opioid receptors. Mol Pharm 50:599-602.
Liang P & Pardee AB 1992: Differential Display of eukaryotic messenger RNA by means of the polymerase chain reaction. Science 257:967-971.
Methner A, Hermey G, Schinke B, Hermanns-Borgmeyer I (1997): A novel G Protein-coupled receptor with homology to neuropeptide and chemoattractant receptors expressed during bone development. Biochem Biophys Res Comm 233: 336-342.
Mohit AA, Martin JH & Miller CA 1995: p493F12 Kinase: a novel MAP kinase expressed in a subset of neurons in the human nervous system. Neuron 14: 67-78.
Poirier GM-C, Pyati J, Wan JS, Erlander MG 1997: Screening differentially expressed cDNA clones obtained by differential display using amplified RNA. Nucleic Acids Research 25: 913-914.
Sambrook J, Fritsch EF & Maniatis T 1989: Molecular Cloning: A Laboratory Manual. Second Edition. Cold Spring Harbor Laboratory Press, Cold Spring Harbor.
Sompayrac L, Jane S, Bum TC, Tenen DG & Danna KJ 1995: Overcoming limitations of the mRNA differential display technique. Nucleic Acids Research 23: 4738-4739.
Tal M 1996: A novel antioxidant alleviates heat hyperalgesia in rats with an experimental painful neuropathy. Neurreport 7: 1382-1384.
Tölle TR (1997): Chronischer Schmerz. In: Klinische Neurobiologie, Herdergen T, Tölle TR, Bähr M (Hrsg.): S. 307-336; Spektrum Verlag, Heidelberg.
U.S.Patent 5.262.311
Velculescu VE, Zhang L, Vogelstein B, Kinzler KW (1995): Serial analysis of gene expression. Science 270: 484-487.
Wan JS, Sharp JS et al. (1996): Cloning differentially expressed mRNAs Nature Biotech 14:1685-1691.
Watson JB & Margulies JE (1993) Differential cDNA screening strategies to identify novel stage-specific proteins in the developing mammalian brain. Dev Neurosci 15: 77-86.
Wilks AF (1989) Two putative protein-tyrosine kinases identified by application of the polymerase chain reaction. Poc Natl Acad Sci USA 86: 1603-1607.
Woolf CJ, Shortland P, Coggeshall RE 1992: Peripheral nerve injury triggers central sprouting of myelinated afferents. Nature 355:75-78.
Zimmermann, M & Herdegen, T (1996): Plasticity of the nervous system at the systemic, cellular and molecular levels: a mechanism of chronic pain and hyperalgesia. Progr Brain Res 110: 233-259

## Patentansprüche

1. Verfahren zur Auffindung schmerzregulierender Substanzen, d.h. Substanzen, die die Wahrnehmung von Schmerz direkt oder indirekt beeinflussen, mit folgenden Verfahrensschritten
(a) Inkubation einer zu testenden Substanz unter geeigneten Bedingungen mit einer Zelle und/oder einer Präparation aus einer solchen Zelle, die das Protein PIM-1-Kinase oder PIM-3-Kinase und/oder ein Protein gemäss einer der Abbildungen 1b), 1d), 1f), 2d) oder 2f) und/oder ein zu einem dieser vorgenannten Proteine zu mindestens 90 % ähnliches Protein und/oder ein Protein, für das ein Polynukleotid gemäss einer der Abbildungen 1a), 1c), 1e), 2a), 2c) oder 2e) oder ein dazu zu mindestens 90 % ähnliches Polynukleotid kodiert, und/oder ein Protein, das durch eine Nukleinsäure kodiert wird, die unter stringenten Bedingungen an ein Polynukleotid gemäss einer der Abbildungen 1a), 1c), 1e), 2a), 2c) oder 2e) oder deren Antisense Polynukleotide binden,
(b) Messung der Bindung der Testsubstanz an dem von der Zelle synthetisierten Protein oder Messung mindestens eines der durch die Bindung der Testsubstanz an das Protein veränderten funktionellen Parameter über Messung der Regulation, Hemmung und/oder Aktivierung von Rezeptoren, Ionenkanälen und/oder Enzymen, insbesondere über Messung der Veränderung der Genexpression, des Ionenmilieus, des pH oder des Membranpotentials, über Veränderung der Enzymaktivität oder der Konzentration der 2^{nd} messenger, oder über Messung der Bindung über die Verdrängung eines bekannten markierten Liganden des Proteins und/oder über die daran gebundene Aktivität einer markierten Testsubstanz.

2. Verfahren gemäss Anspruch 1, **dadurch gekennzeichnet, dass** die Zelle vor dem Schritt (a) gentechnisch manipuliert wird.

3. Verfahren gemäss Anspruch 2, **dadurch gekennzeichnet, dass** die gentechnische Manipulation die Messung mindestens eines der durch die Testsubstanz veränderten funktionellen Parameter erlaubt.

4. Verfahren gemäss Anspruch 3, **dadurch gekennzeichnet, dass** durch die gentechnische Manipulation eine in der Zelle nicht endogen exprimierte Form eines G-Proteins exprimiert oder ein Reportergen eingeführt wird.

5. Verfahren gemäss einem der Ansprüche 2-4, **dadurch gekennzeichnet, dass** die Zelle gentechnisch so manipuliert wird, dass die Zelle mindestens ein Polynukleotid gemäss einer der Abbildungen 1a), 1c), 1e), 2a), 2c) oder 2e) oder ein dazu zu mindestens 90 % ähnliches Polynukleotid enthält.

6. Verfahren gemäss Anspruch 5, **dadurch gekennzeichnet, dass** das Polynukleotid in einem rekombinanten DNA-Konstrukt enthalten ist.

7. Verfahren gemäss einem der Ansprüche 2 bis 6, **dadurch gekennzeichnet, dass** die Zelle nach der gentechnischen Manipulation gemäss Anspruch 2 und vor dem Schritt (a) gemäss Anspruch 1 unter Bedingungen, die eine Expression erlauben, kultiviert wird, gegebenenfalls unter Selektionsdruck.

8. Verfahren gemäss einem der Ansprüche 1 bis 7, **dadurch gekennzeichnet, dass** die Zelle eine Amphibienzelle, Bakterienzelle, Hefezelle, Insektenzelle oder eine immortalisierte oder native Säugetierzelle ist.

9. Verfahren gemäss einem der Ansprüche 1-8, **dadurch gekennzeichnet, dass** das Protein in den Schritten (a) und (b) ausgewählt ist aus:
- der PIM-1-Kinase,
- einem Protein, für das ein Polynukleotid gemäss einer der Abbildungen 1a), 1c) oder 1e) oder ein dazu zu mindestens 90 %, vorzugsweise 95%, insbesondere 97 %, ähnliches Polynukleotid kodiert,
- einem Protein mit einer Aminosäuresequenz gemäss einer der Abbildungen 1b), 1d), 1f), oder einem dazu zu mindestens 90 %, vorzugsweise 95 %, insbesondere 97 %, ähnlichem Protein und/oder
- einem Protein, das durch eine Nukleinsäure kodiert wird, die unter stringenten Bedingungen an ein Polynukleotid gemäss einer der Abbildungen 1a), 1c) oder 1e) oder deren Antisense Polynukleotide bindet,

10. Verfahren gemäss einem der Ansprüche 1 bis 9, **dadurch gekennzeichnet, dass** in einem Teilverfahren das Protein in den Schritten (a) und (b) ausgewählt ist aus:
- der PIM-1-Kinase,
- einem Protein, für das ein Polynukleotid gemäss einer der Abbildungen 1a), 1c) oder 1e) oder ein dazu zu mindestens 90 %, vorzugsweise 95 %, insbesondere 97 %, ähnliches Polynukleotid kodiert,
- einem Protein mit einer Aminosäuresequenz gemäss einer der Abbildungen 1b), 1d), 1f), oder einem dazu zu mindestens 90 %, vorzugsweise 95 %, insbesondere 97 %, ähnlichem Protein und/oder
- einem Protein, das durch eine Nukleinsäure kodiert wird, die unter stringenten Bedingungen an ein Polynukleotid gemäss einer der Abbildungen 1a), 1c) oder 1e) oder deren Antisense Polynukleotide bindet,
und in einem anderen Teilverfahren das Protein in den Schritten (a) und (b) ausgewählt ist aus:
- der PIM-2-Kinase,
oder
- der PIM-3-Kinase,
- einem Protein, für das ein Polynukleotid gemäss einer der Abbildungen 2a), 2c) oder 2e) oder ein dazu zu mindestens 90 %, vorzugsweise 95 %, insbesondere 97 %, ähnliches Polynukleotid kodiert,
- einem Protein mit einer Aminosäuresequenz gemäss einer der Abbildungen 2d), 2f), oder einem dazu zu mindestens 90 %, vorzugsweise 95 %, insbesondere 97 %, ähnlichem Protein und/oder
- einem Protein, das durch eine Nukleinsäure kodiert wird, die unter stringenten Bedingungen an ein Polynukleotid gemäss einer der Abbildungen 2a), 2c) oder 2e) oder deren Antisense Polynukleotide bindet,
und die Ergebnisse aus Schritt b) der Teilverfahren differentiell verglichen werden.

## Claims

1. A method for screening for pain regulating substances, i.e. substances which affect the perception of pain directly or indirectly, comprising the following steps:
(a) incubating under suitable conditions a substance to be tested, with a cell and/or a preparation of such a cell having synthesized the protein PIM-1-kinase or PIM-3-kinase, and/or a protein according to any one of Figures 1b), 1d), 1f), 2d) or 2f), and/or a protein having a similarity of at least 90 % to any one of the aforementioned proteins, and/or a protein encoded by a polynucleotide according to any one of Figures 1a), 1c), 1e), 2a), 2c) or 2e) or a polynucleotide having a similarity of at least 90 % thereto, and/or a protein encoded by a nucleic acid, which bind to a polynucleotide according to any one of Figures 1a), 1c), 1e), 2a), 2c) or 2e) or their antisense polynucleotides under stringent conditions,
(b) measuring the binding of the test substance to the protein synthesized by the cell or measuring at least one of the functional parameters altered by the binding of the test substance to the protein via measuring the regulation, inhibition, and/or activation of receptors, ion channels and/or enzymes, especially via measuring the alteration of the gene expression, of the ion environment, of the pH or of the membrane potential, via alteration of the enzyme activity or concentration of the 2^{nd} messengers, or via measuring the binding via the displacement of a known labelled ligand of the protein and/or via the activity of a labelled test substance bound thereto.

2. The method according to claim 1, **characterized in that** the cell is genetically manipulated prior to step (a).

3. The method according to claim 2, **characterized in that** the genetic manipulation allows for the measurement of at least one of the functional parameters altered by the test substance.

4. The method according to claim 3, **characterized in that** a form of a G-protein, the form being not endogenously expressed in the cell, or a reporter gene is introduced by the genetic manipulation.

5. The method according to any one of claims 2 to 4, **characterized in that** the cell is genetically manipulated such that the cell contains a least one polynucleotide according to any one of Figures 1a), 1c), 1e), 2a), 2c) or 2e) or a polynucleotide having a similarity of at least 90 % thereto.

6. The method according to claim 5, **characterized in that** the polynucleotide is contained in a recombinant DNA construct.

7. The method according to any one of claims 2 to 6, **characterized in that** the cell is cultivated, after the genetic manipulation according to claim 2 and prior to step (a) according to claim 1, under conditions permissive to expression, optionally under selection pressure.

8. The method according to any one of claims 1 to 7, **characterized in that** the cell is an amphibian cell, bacterial cell, yeast cell, insect cell or an immortalized or native mammalian cell.

9. The method according to any one of claims 1 to 8, **characterized in that** the protein in steps (a) and (b) is selected from:
- the PIM-1-kinase,
- a protein encoded by a polynucleotide according to any one of Figures 1a), 1c) or 1e) or a polynucleotide having a similarity thereto of at least 90 %, preferably 95 %, especially 97 %,
- a protein having an amino acid sequence according to any one of Figures 1b), 1d), 1f), or a protein having a similarity thereto of at least 90 %, preferably 95 %, especially 97 %, and/or
- a protein encoded by a nucleic acid which binds to a polynucleotide according to any one of Figures 1a), 1c), or 1e) or their antisense polynucleotides under stringent conditions.

10. A method according to any one of claims 1 to 9, **characterized in that** in a partial method the protein in steps (a) and (b) is selected from:
- the PIM-1-kinase,
- a protein encoded by a polynucleotide according to any one of Figures 1a), 1c) or 1e), or a polynucleotide having a similarity thereto of at least 90 %, preferably 95 %, especially 97 %,
- a protein having an amino acid sequence according to any one of Figures 1b), 1d), 1f), or a protein having a similarity thereto of at least 90 %, preferably 95 %, especially 97 %, and/or
- a protein encoded by a nucleic acid, which binds to a polynucleotide according to any one of Figures 1a), 1c) or 1e) or their antisense polynucleotides under stringent conditions,
and in an other partial method the protein in steps (a) and (b) is selected from:
- the PIM-2-kinase,
or
- the PIM-3-kinase,
- a protein encoded by a polynucleotide according to any one of Figures 2a), 2c) or 2e), or a polynucleotide having a similarity thereto of at least 90 %, preferably 95 %, especially 97 %,
- a protein having an amino acid sequence according to any one of Figures 2d), 2f), or a protein having a similarity thereto of at least 90 %, preferably 95 %, especially 97 %, and/or
- a protein encoded by a nucleic acid, which binds to a polynucleotide according to any one of Figures 2a), 2c), or 2e) or their antisense polynucleotides under stringent conditions,
and the results of step b) of the partial methods are differentially compared.

## Revendications

1. Méthode de criblage de substances régulatrices de la douleur, c'est-à-dire de substances qui influent directement ou indirectement sur la perception de la douleur, méthode comprenant les étapes suivantes
(a) incubation d'une substance à éprouver, dans des conditions convenables, avec une cellule et/ou une préparation obtenue à partir d'une cellule qui a synthétisé la protéine PIM-1-kinase ou PIM-3-kinase et/ou une protéine selon l'une des figures 1b), 1d), 1f), 2d) ou 2f) et/ou une protéine semblable à 90 % au moins à l'une des protéines mentionnées ci-dessus et/ou une protéine qui est codée par un polynucléotide selon l'une des figures 1a), 1c), 1e), 2a), 2c) ou 2e) ou par un polynucléotide qui y est semblable à 90 % au moins, et/ou une protéine qui est codée par un acide nucléique, qui se lient dans des conditions stringentes à un polynucléotide selon l'une des figures 1a), 1c), 1e), 2a), 2c) ou 2e) ou à leurs polynucléotides antisens,
(b) mesure de la liaison de la substance d'essai à la protéine synthétisée par la cellule ou mesure de l'un au moins des paramètres fonctionnels modifiés par la liaison de la substance d'essai à la protéine par l'intermédiaire de la mesure de la régulation, de l'inhibition et/ou de l'activation de récepteurs, de canaux ioniques et/ou d'enzymes, en particulier par l'intermédiaire de la mesure de la variation de l'expression génique, du milieu ionique, du pH ou du potentiel de membrane, par l'intermédiaire de la variation de l'activité enzymatique ou de la concentration des seconds messagers, ou mesure de la liaison par l'intermédiaire du déplacement d'un ligand marqué connu de la protéine et/ou par l'intermédiaire de l'activité d'une substance d'essai marquée qui y est liée.

2. Méthode suivant la revendication 1, **caractérisée en ce que** la cellule est traitée par manipulation génétique avant l'étape (a).

3. Méthode suivant la revendication 2, **caractérisée en ce que** la manipulation génétique permet la mesure d'au moins l'un des paramètres fonctionnels modifiés par la substance d'essai.

4. Méthode suivant la revendication 3, **caractérisée en ce qu'**une forme d'expression non endogène d'une protéine G est exprimée dans la cellule ou bien un gène rapporteur est introduit par la manipulation génétique.

5. Méthode suivant l'une des revendications 2 à 4, **caractérisée en ce que** la cellule est traitée par manipulation génétique de façon telle qu'elle contient au moins un polynucléotide selon l'une des figures 1a), 1c), 1e), 2a), 2c) ou 2e) ou un polynucléotide qui y est semblable à 90 % au moins.

6. Méthode suivant la revendication 5, **caractérisée en ce que** le polynucléotide est contenu dans un produit d'assemblage par recombinaison d'ADN.

7. Méthode suivant l'une des revendications 2 à 6, **caractérisée en ce que** la cellule est cultivée après la manipulation génétique selon la revendication 2, et avant l'étape (a) selon la revendication 1 dans des conditions qui permettent une expression, le cas échéant sous pression de sélection.

8. Méthode suivant l'une des revendications 1 à 7, **caractérisée en ce que** la cellule est une cellule d'amphibien, une cellule de bactérie, une cellule de levure, une cellule d'insecte ou une cellule immortalisée ou native de mammifère.

9. Méthode suivant l'une des revendications 1 à 8, **caractérisée en ce que** la protéine, dans les étapes (a) et (b), est choisie entre :
- la PIM-1-kinase,
- une protéine qui est codée par un polynucléotide selon l'une des figures 1a), 1c) ou 1e) ou par un polynucléotide qui y est semblable à au moins 90 %, avantageusement à 95 %, notamment à 97 %,
- une protéine ayant une séquence d'acides aminés selon l'une des figures 1b), 1d), 1f) ou une protéine qui y est semblable à au moins 90 %, avantageusement à 95 %, notamment à 97 % et/ou
- une protéine qui est codée par un acide nucléique qui se lie dans des conditions stringentes à un polynucléotide selon l'une des figures 1a), 1c) ou 1e) ou leurs polynucléotides antisens.

10. Méthode suivant l'une des revendications 1 à 9, **caractérisée en ce que** dans une méthode partielle, la protéine est choisie dans les étapes (a) et (b) entre :
- la PIM-1-kinase,
- une protéine qui est codée par un polynucléotide selon l'une des figures 1a), 1c) ou 1e) ou par un polynucléotide qui y est semblable à au moins 90 %, avantageusement à 95 %, notamment à 97 %,
- une protéine ayant une séquence d'acides aminés selon l'une des figures 1b), 1d), 1f) ou une protéine qui y est semblable à au moins 90 %, avantageusement à 95 %, notamment à 97 % et/ou
- une protéine qui est codée par un acide nucléique qui se lie dans des conditions stringentes à un polynucléotide selon l'une des figures 1a), 1c) ou 1e) ou leurs polynucléotides antisens,
et dans une autre méthode partielle, la protéine dans les étapes (a) et (b) est choisie entre
- la PIM-2-kinase, ou
- la PIM-3-kinase
- une protéine qui est codée par un polynucléotide selon l'une des figures 2a), 2c) ou 2e) ou un polynucléotide qui y est semblable à au moins 90 %, avantageusement à 95 %, notamment à 97 %,
- une protéine ayant une séquence d'acides aminés selon l'une des figures 2d), 2f) ou une protéine qui y est semblable à au moins 90 %, avantageusement à 95 %, notamment à 97 % et/ou
- une protéine qui est codée par un acide nucléique qui se lie dans des conditions stringentes à un polynucléotide selon l'une des figures 2a), 2c) ou 2e) ou leurs polynucléotides antisens,
et les résultats de l'étape b) des méthodes partielles sont comparés de façon différentielle.


### SFQUENZPROTOKOLL

<110> GRÜNENTHAL GmbH, Aachen, Deutschland
<120> Screeningverfahren mit PIM1-Kinase oder PIM3-Kinase
<130> GRA 3064-PCT
<140> PCT/EP02/05234
   <141> 2002-05-13
<150> DE 101 23 055.9
   <151> 2001-05-11
<160> 11
<170> PatentIn Ver. 2.1
<210> 1
   <211> 2623
   <212> DNA
   <213> Homo sapiens
<400> 1
   gaggaggccc gagaggagtc ggtggcagcg gcggcggcgg gaccggcagc agcagcagca 60
   gcagcagcag caaccactag cctcctgccc cgcggcgttg cgacgagccc cacgagccgc 120
   tcaccccgcc gttctcagcg ctgcccgacc ccgctggcgc gcctcccgcc gcagtcccgg 180
   cagcgcctca gttgtcctcc gactcgccct cggccttcgc gcagcgcagc acagccgcac 240
   gcaccgcagc acagcacagc acagcccagg catagcttcg gcacagcccc ggctccggct 300
   cctgcggcag ctcctctggc acgtccctgc gccgacattc tggaggttgg atgctcttgt 360
   ccaaaatcaa ctcgcttgcc cacctgcgcg ccgcgccctg caacgacctg cacgccacca 420
   agctggcgcc cggcaaggag aaggagcccc tggagtcgca gtaccaggtg ggcccgctac 480
   tgggcagcgg cggcttcggc tcggtctact caggcatccg cgtctccgac aacttgccgg 540
   tggccatcaa acacgtggag aaggaccgga tttccgactg gggagagctg cctaatggca 600
   ctcgagtgcc catggaagtg gtcctgctga agaaggtgag ctcgggtttc tccggcgtca 660
   ttaggctcct ggactggttc gagaggcccg acagtttcgt cctgatcctg gagaggcccg 720
   agccggtgca agatctcttc gacttcatca cggaaagggg agccctgcaa gaggagctgg 780
   cccgcagctt cttctggcag gtgctggagg ccgtgcggca ctgccacaac tgcggggtgc 840
   tacaccgcga catcaaggac gaaaacatcc ttatcgacct caatcgcggc gagctcaagc 900
   tcatcgactt cgggtcgggg gcgctgctca aggacaccgt ctacacggac ttcgatggga 960
   cccgagtgta tagccctcca gagtggatcc gctaccatcg ctaccatggc aggtcggcgg 1020
   cagtctggtc cctggggatc ctgctgtatg atatggtgtg tggagatatt cctttcgagc 1080
   atgacgaaga gatcatcagg ggccaggttt tcttcaggca gagggtctct tcagaatgtc 1140
   agcatctcat tagatggtgc ttggccctga gaccatcaga taggccaacc ttcgaagaaa 1200
   tccagaacca tccatggatg caagatgttc tcctgcccca ggaaactgct gagatccacc 1260
   tccacagcct gtcgccgggg cccagcaaat agcagccttt ctggcaggtc ctcccctctc 1320
   ttgtcagatg cccgagggag gggaagcttc tgtctccagc ttcccgagta ccagtgacac 1380
   gtctcgccaa gcaggacagt gcttgataca ggaacaacat ttacaactca ttccagatcc 1440
   caggcccctg gaggctgcct cccaacagtg gggaagagtg actctccagg ggtcctaggc 1500
   ctcaactcct cccatagata ctctcttctt ctcataggtg tccagcattg ctggactctg 1560
   aaatatcccg ggggtggggg gtgggggtgg gcagaaccct gccaatggaa ctctttcttc 1620
   atcatgagtt ctgctgaatg ccgcgatggg tcaggtaggg gggaaacagg ttgggatggg 1680
   ataggactag cacattttaa gtccctgtca cctcttccga ctctttctga gtgccttctg 1740
   tggggactcc ggctgtgctg ggagaaatac ttgaacttgc ctcttttacc tgctgcttct 1800
   ccaaaaatct gcctgggttt tgttccctat ttttctctcc tgtcctccct caccccctcc 1860
   ttcatatgaa aggtgccatg gaagaggcta cagggccaaa cgctgagcca cctgcccttt 1920
   tttctgcctc ctttagtaaa actccgagtg aactggtctt cctttttggt ttttacttaa 1980
   ctgtttcaaa gccaagacct cacacacaca aaaaaatgca caaaccaagc aatcaacaga 2040
   aaagctgtaa atgtgtgtac agttggcatg gtagtataca aaaagattgt agtggatcta 2100
   atttttaaga aattttgcct ttaagttatt ttacctgttt ttgtttcttg ttttgaaaga 2160
   tgcgcattct aacctggagg tcaatgttat gtatttattt atttatttat ttggttccct 2220
   tcctattcca agcttccata gctgctgccc tagttttctt tcctcctttc ctcctctgac 2280
   ttggggacct tttgggggag ggctgcgacg cttgctctgt ttgtggggtg acgggactca 2340
   ggcgggacag tgctgcagct ccctggcttc tgtggggccc ctcacctact tacccaggtg 2400
   ggtcccggct ctgtgggtga tgggaggggc cattgctgac tgtgtatata ggataattat 2460
   gaaacacagt tctggatggt gtgccttcca gatcctctct ggggctgtgt tttgagcagc 2520
   aggtagcctg ctggttttat ctgagtgaaa tactgtacag gggaataaaa gagatcttat 2580
   ttttttttta tacttgcgtt tggaataaaa accctttggc ttt                    2623
<210> 2
   <211> 313
   <212> PRT
   <213> Homo sapiens
<400> 2
   Met Leu Leu Ser Lys Ile Asn Ser Leu Ala His Leu Arg Ala Ala Pro
    1               5                   10                  15
   Cys Asn Asp Leu His Ala Thr Lys Leu Ala Pro Gly Lys Glu Lys Glu
               20                  25                  30
   Pro Leu Glu Ser Gln Tyr Gln Val Gly Pro Leu Leu Gly Ser Gly Gly
           35                  40                  45
   Phe Gly Ser Val Tyr Ser Gly Ile Arg Val Ser Asp Asn Leu Pro Val
       50                  55                  60
   Ala Ile Lys His Val Glu Lys Asp Arg Ile Ser Asp Trp Gly Glu Leu
   65                  70                  75                  80
   Pro Asn Gly Thr Arg Val Pro Met Glu Val Val Leu Leu Lys Lys Val
                   85                  90                  95
   Ser Ser Gly Phe Ser Gly Val Ile Arg Leu Leu Asp Trp Phe Glu Arg
               100                 105                 110
   Pro Asp Ser Phe Val Leu Ile Leu Glu Arg Pro Glu Pro Val Gln Asp
   115 120 125
   Leu Phe Asp Phe Ile Thr Glu Arg Gly Ala Leu Gln Glu Glu Leu Ala
   130 135 140
   Arg Ser Phe Phe Trp Gln Val Leu Glu Ala Val Arg His Cys His Asn
   145 150 155 160
   Cys Gly Val Leu His Arg Asp Ile Lys Asp Glu Asn Ile Leu Ile Asp
   165 170 175
   Leu Asn Arg Gly Glu Leu Lys Leu Ile Asp Phe Gly Ser Gly Ala Leu
   180 185 190
   Leu Lys Asp Thr Val Tyr Thr Asp Phe Asp Gly Thr Arg Val Tyr Ser
   195 200 205
   Pro Pro Glu Trp Ile Arg Tyr His Arg Tyr His Gly Arg Ser Ala Ala
   210 215 220
   Val Trp Ser Leu Gly Ile Leu Leu Tyr Asp Met Val Cys Gly Asp Ile
   225 230 235 240
   Pro Phe Glu His Asp Glu Glu Ile Ile Arg Gly Gln Val Phe Phe Arg
   245 250 255
   Gln Arg Val Ser Ser Glu Cys Gln His Leu Ile Arg Trp Cys Leu Ala
   260 265 270
   Leu Arg Pro Ser Asp Arg Pro Thr Phe Glu Glu Ile Gln Asn His Pro
   275 280 285
   Trp Met Gln Asp Val Leu Leu Pro Gln Glu Thr Ala Glu Ile His Leu
   290 295 300
   His Ser Leu Ser Pro Gly Pro Ser Lys
   305 310
<210> 3
   <211> 1302
   <212> DNA
   <213> Rattus norvegicus
<400> 3
   gggatgctct tgtccaagat caactccctg gcccacctgc gcgcagcccc ttgcaacgac 60
   ctgcacgcca acaagctggc gccgggcaaa gagaaggagc ccctggagtc gcagtaccag 120
   gtgggcccgc tgttgggcag cggtggcttc ggctcggtct actcgggcat ccgcgtcgcc 180
   gacaacttgc cggtggccat caagcacgtg gagaaggacc ggatttccga ctggggggaa 240
   ctgcccaacg gcacccgagt gcccatggaa gtggtcctgc tgaagaaggt gagctcgggc 300
   ttctcgggcg tcattagact tctggactgg ttcgagaggc ccgatagttt cgtgctgatc 360
   ctggagaggc ccgaacccgt gcaagacctc ttcgacttca tcaccgagcg aggagccctc 420
   caggaggagc tggcccggag cttcttctgg caggtgctgg aggccgtgcg gcattgccac 480
   aactgcgggg ttctccaccg cgacatcaag gacgagaaca tcttaatcga cctgaaccgc 540
   ggcgaactca aactcatcga cttcgggtcg ggggcgctgc tcaaggacac agtctacacg 600
   gactttgacg gaacccgagt gtacagtcct ccagagtgga ttcgctacca tcgctaccac 660
   ggcaggtcgg ctgctgtttg gtccctgggg atcctgctct atgacatggt ctgcggagat 720
   attccatttg agcacgacga agagatcgtc aagggccaag tgtactttag gcaaagggtc 780
   tcttcagaat gtcaacatct tattagatgg tgcctgtccc tgagaccatc ggaccggccc 840
   tcctttgaag aaatccagaa ccatccgtgg atgcaggatg ttctcctgcc ccaggccacc 900
   gccgagattc atctgcacag cctgtcacca tcacccagca aatagcagcc attctgtcag 960
   accctccagg gaagagagag cttgtctgct ggcctccaac aggaccctgc tctacgatgc 1020
   agggacagaa atgacaactc attccaggct ccggggtccc tggagcaacc tccctcaagg 1080
   agaagagact agttcactcg tcctggaccc cgctttgccc ctcacagact cagtggcgtc 1140
   cagtgtggct ggcgtccgca gagtcccggg tgttgggggg ggaggtggga gtgggtcaga 1200
   gccctgtcat ggaactttag tcaccatgga gactgtgggt caccaagatg ggccagggta 1260
   ggggaaaaac atttgggggg tgggattaaa aactagcacc at                    1302
<210> 4
   <211> 313
   <212> PRT
   <213> Rattus norvegicus
<400> 4
   Met Leu Leu Ser Lys Ile Asn Ser Leu Ala His Leu Arg Ala Ala Pro
   1               5                   10                  15
   Cys Asn Asp Leu His Ala Asn Lys Leu Ala Pro Gly Lys Glu Lys Glu
               20                  25                  30
   Pro Leu Glu Ser Gln Tyr Gln Val Gly Pro Leu Leu Gly Ser Gly Gly
           35                  40                  45
   Phe Gly Ser Val Tyr Ser Gly Ile Arg Val Ala Asp Asn Leu Pro Val
       50                  55                  60
   Ala Ile Lys His Val Glu Lys Asp Arg Ile Ser Asp Trp Gly Glu Leu
   65                  70                  75                  80
   Pro Asn Gly Thr Arg Val Pro Met Glu Val Val Leu Leu Lys Lys Val
                   85                  90                  95
   Ser Ser Gly Phe Ser Gly Val Ile Arg Leu Leu Asp Trp Phe Glu Arg
   100 105 110
   Pro Asp Ser Phe Val Leu Ile Leu Glu Arg Pro Glu Pro Val Gln Asp
   115 120 125
   Leu Phe Asp Phe Ile Thr Glu Arg Gly Ala Leu Gln Glu Glu Leu Ala
   130 135 140
   Arg Ser Phe Phe Trp Gln Val Leu Glu Ala Val Arg His Cys His Asn
   145 150 155 160
   Cys Gly Val Leu His Arg Asp Ile Lys Asp Glu Asn Ile Leu Ile Asp
   165 170 175
   Leu Asn Arg Gly Glu Leu Lys Leu Ile Asp Phe Gly Ser Gly Ala Leu
   180 185 190
   Leu Lys Asp Thr Val Tyr Thr Asp Phe Asp Gly Thr Arg Val Tyr Ser
   195 200 205
   Pro Pro Glu Trp Ile Arg Tyr His Arg Tyr His Gly Arg Ser Ala Ala
   210 215 220
   Val Trp Ser Leu Gly Ile Leu Leu Tyr Asp Met Val Cys Gly Asp Ile
   225 230 235 240
   Pro Phe Glu His Asp Glu Glu Ile Val Lys Gly Gln Val Tyr Phe Arg
   245 250 255
   Gln Arg Val Ser Ser Glu Cys Gln His Leu Ile Arg Trp Cys Leu Ser
   260 265 270
   Leu Arg Pro Ser Asp Arg Pro Ser Phe Glu Glu Ile Gln Asn His Pro
   275 280 285
   Trp Met Gln Asp Val Leu Leu Pro Gln Ala Thr Ala Glu Ile His Leu
   290 295 300
   His Ser Leu Ser Pro Ser Pro Ser Lys
   305 310
<210> 5
   <211> 942
   <212> DNA
   <213> Mus musculus
<400> 5
   atgctcctgt ccaagatcaa ctccctggcc cacctgcgcg cccgcccctg caacgacctg 60
   cacgccacca agctggcgcc gggcaaagag aaggagcccc tggagtcgca gtaccaggtg 120
   ggcccgctgt tgggcagcgg tggcttcggc tcggtctact ctggcatccg cgtcgccgac 180
   aacttgccgg tggccattaa gcacgtggag aaggaccgga tttccgattg gggagaactg 240
   cccaatggca cccgagtgcc catggaagtg gtcctgttga agaaggtgag ctcggacttc 300
   tcgggcgtca ttagacttct ggactggttc gagaggcccg atagtttcgt gctgatcctg 360
   gagaggcccg aaccggtgca agacctcttc gactttatca ccgaacgagg agccctacag 420
   gaggacctgg cccgaggatt cttctggcag gtgctggagg ccgtgcggca ttgccacaac 480
   tgcggggttc tccaccgcga catcaaggac gagaacatct taatcgacct gagccgcggc 540
   gaaatcaaac tcatcgactt cgggtcgggg gcgctgctca aggacacagt ctacacggac 600
   tttgatggga cccgagtgta cagtcctcca gagtggattc gctaccatcg ctaccacggc 660
   aggtcggcag ctgtctggtc ccttgggatc ctgctctatg acatggtctg cggagatatt 720
   ccgtttgagc acgatgaaga gatcatcaag ggccaagtgt tcttcaggca aactgtctct 780
   tcagagtgtc agcaccttat taaatggtgc ctgtccctga gaccgtcaga tcggccctcc 840
   tttgaagaaa tccggaacca tccgtggatg cagggtgacc tcctgcccca ggcagcttct 900
   gagatccatc tgcacagtct gtcaccggga tccagcaagt ag                   942
<210> 6
   <211> 313
   <212> PRT
   <213> Mus musculus
<400> 6
   Met Leu Leu Ser Lys Ile Asn Ser Leu Ala His Leu Arg Ala Arg Pro
   1               5                   10                  15
   Cys Asn Asp Leu His Ala Thr Lys Leu Ala Pro Gly Lys Glu Lys Glu
               20                  25                  30
   Pro Leu Glu Ser Gln Tyr Gln Val Gly Pro Leu Leu Gly Ser Gly Gly
           35                  40                  45
   Phe Gly Ser Val Tyr Ser Gly Ile Arg Val Ala Asp Asn Leu Pro Val
       50                  55                  60
   Ala Ile Lys His Val Glu Lys Asp Arg Ile Ser Asp Trp Gly Glu Leu
   65                  70                  75                  80
   Pro Asn Gly Thr Arg Val Pro Met Glu Val Val Leu Leu Lys Lys Val
                   85                  90                  95
   Ser Ser Asp Phe Ser Gly Val Ile Arg Leu Leu Asp Trp Phe Glu Arg
               100                 105                 110
   Pro Asp Ser Phe Val Leu Ile Leu Glu Arg Pro Glu Pro Val Gln Asp
   115 120 125
   Leu Phe Asp Phe Ile Thr Glu Arg Gly Ala Leu Gln Glu Asp Leu Ala
   130 135 140
   Arg Gly Phe Phe Trp Gln Val Leu Glu Ala Val Arg His Cys His Asn
   145 150 155 160
   Cys Gly Val Leu His Arg Asp Ile Lys Asp Glu Asn Ile Leu Ile Asp
   165 170 175
   Leu Ser Arg Gly Glu Ile Lys Leu Ile Asp Phe Gly Ser Gly Ala Leu
   180 185 190
   Leu Lys Asp Thr Val Tyr Thr Asp Phe Asp Gly Thr Arg Val Tyr Ser
   195 200 205
   Pro Pro Glu Trp Ile Arg Tyr His Arg Tyr His Gly Arg Ser Ala Ala
   210 215 220
   Val Trp Ser Leu Gly Ile Leu Leu Tyr Asp Met Val Cys Gly Asp Ile
   225 230 235 240
   Pro Phe Glu His Asp Glu Glu Ile Ile Lys Gly Gln Val Phe Phe Arg
   245 250 255
   Gln Thr Val Ser Ser Glu Cys Gln His Leu Ile Lys Trp Cys Leu Ser
   260 265 270
   Leu Arg Pro Ser Asp Arg Pro Ser Phe Glu Glu Ile Arg Asn His Pro
   275 280 285
   Trp Met Gln Gly Asp Leu Leu Pro Gln Ala Ala Ser Glu Ile His Leu
   290 295 300
   His Ser Leu Ser Pro Gly Ser Ser Lys
   305 310
<210> 7
   <211> 2133
   <212> DNA
   <213> Rattus norvegicus
<400> 7
   cgctcggcca gctgccgtct acgggcttcc gcgcggccac cgggcaactg cgccgcgcgg 60
   ctgccccact gagcgctcgg cctcggggcc gtgggatccg ccgcgctgtc tgcggtcagg 120
   aagaccgccc tcccgcgtcc gtgccggacg ggtcagaggc ggcgccgcac gcgaggccac 180
   ccgcgatgct gctgtccaag ttcggctccc tggcgcacct ctgcgggcct ggcggcgtgg 240
   accacctccc agtgaagatc ctacagccag ccaaggcgga caaggagagc ttcgagaagg 300
   tgtaccaggt gggcgccgtg ctcggcagcg gcggcttcgg cacggtctac gcgggcagcc 360
   gcatcgccga cggactcccg gtggctgtga agcacgtggt gaaggagcgg gtgaccgagt 420
   ggggcagtct cggcggaatg gccgtgcccc tggaggtggt gctgctgcgc aaggtgggcg 480
   cggcgggcgg cgcgcgcggc gtcatccgcc tgctggactg gttcgagcgg cccgacggct 540
   tcctgctggt gctggagcga cccgagccgg cacaggacct cttcgacttc atcactgaac 600
   gcggcgccct ggacgagcct ctggctcgtc gcttcttcgc gcaggtgctc gccgctgtgc 660
   ggcactgcca caattgtggg gtcgtgcacc gcgacatcaa ggacgagaac ctgctggtgg 720
   acttgcgctc gggcgagctg aagctcatcg acttcggctc gggcgcggtg ctcaaggaca 780
   cggtctacac tgactttgat ggcacccgtg tgtacagccc cccagagtgg atccggtatc 840
   atcgatatca cgggcggtct gccactgtgt ggtctctggg tgtactgctc tacgacatgg 900
   tgtgtgggga cattcccttt gagcaggatg aggagatctt gcgcggcagg ctctttttcc 960
   ggaggagggt ctccccagag tgccagcagc ttattgagtg gtgtctctcc ctgcggccct 1020
   cagagaggcc ctcgctggac caaattgctg cccatccctg gatgctgggg acagagggca 1080
   gcgttccaga gaactgtgac cttcggctct gtgccctgga tactgatgac ggagccagta 1140
   ccacttccag cagtgagagc ttgtgaggag gaggaggggc ctggactcca cactgggggc 1200
   ctgggctcag cctagccagc cctctcccag aatgaacatt ttctgcctgg gatgtctcct 1260
   gcaaaagcag tgacctctga cccctggtga cctttgctct cggcaccggg cctgtttcct 1320
   ttgctttgag tgcctttttg aacgctgctc cacagggcct gggttttctt gagctcttct 1380
   gtccaaagat ggctgcgggc taagcaaggt cccgcctgcc ctgggtggat acttgaaccc 1440
   gagaccctac cctgctgctc catcttgcgg cagccttcct gaccaagtgt gtttgacatg 1500
   gagcgccctg tggtgcccac ctccaaccct ccagtctcct ggtcttcgtc tgggcatgtc 1560
   tgcacaagca atgcaacgct gggccactgc tgcccgcctg cctccctggc accgcacgca 1620
   acgagcgtgc cacggtctct tatttatggt gtgatcaccc tggagggcgc ccctgccctg 1680
   ctggggctat ttattgttta atttatttgc tgaggttact tcctccaagc aaccaccttc 1740
   tccaggcccc tggggtgttc aggaaagcca agggtggccg ttcagtccac agacggcatc 1800
   ctggttcctg cacctgcagt aggtccctaa ccccatgttt gtgggaggag gaatttgtac 1860
   agtggctaat ttaaggggag tgggagaccc tgtcaccctg ggcactctgc gctggggagg 1920
   gggtttaaat tattgacctt gtacagtctg cttgctggct ctgaaagctg gggtggggga 1980
   cagagtctca agcccttaat ttattttagc aactgtgttc tgtgaccctg gtgtgagtag 2040
   gcatcagggg tggggttgta taagttcaaa agtgtgaaat gtctggagat catatttttt 2100
   atacaggtat ttcaattaaa tgttttggta tat                              2133
<210> 8
   <211> 326
   <212> PRT
   <213> Rattus norvegicus
<400> 8
   Met Leu Leu Ser Lys Phe Gly Ser Leu Ala His Leu Cys Gly Pro Gly
   1               5                   10                  15
   Gly Val Asp His Leu Pro Val Lys Ile Leu Gln Pro Ala Lys Ala Asp
               20                  25                  30
   Lys Glu Ser Phe Glu Lys Val Tyr Gln Val Gly Ala Val Leu Gly Ser
   35 40 45
   Gly Gly Phe Gly Thr Val Tyr Ala Gly Ser Arg Ile Ala Asp Gly Leu
   50 55 60
   Pro Val Ala Val Lys His Val Val Lys Glu Arg Val Thr Glu Trp Gly
   65 70 75 80
   Ser Leu Gly Gly Met Ala Val Pro Leu Glu Val Val Leu Leu Arg Lys
   85 90 95
   Val Gly Ala Ala Gly Gly Ala Arg Gly Val Ile Arg Leu Leu Asp Trp
   100 105 110
   Phe Glu Arg Pro Asp Gly Phe Leu Leu Val Leu Glu Arg Pro Glu Pro
   115 120 125
   Ala Gln Asp Leu Phe Asp Phe Ile Thr Glu Arg Gly Ala Leu Asp Glu
   130 135 140
   Pro Leu Ala Arg Arg Phe Phe Ala Gln Val Leu Ala Ala Val Arg His
   145 150 155 160
   Cys His Asn Cys Gly Val Val His Arg Asp Ile Lys Asp Glu Asn Leu
   165 170 175
   Leu Val Asp Leu Arg Ser Gly Glu Leu Lys Leu Ile Asp Phe Gly Ser
   180 185 190
   Gly Ala Val Leu Lys Asp Thr Val Tyr Thr Asp Phe Asp Gly Thr Arg
   195 200 205
   Val Tyr Ser Pro Pro Glu Trp Ile Arg Tyr His Arg Tyr His Gly Arg
   210 215 220
   Ser Ala Thr Val Trp Ser Leu Gly Val Leu Leu Tyr Asp Met Val Cys
   225 230 235 240
   Gly Asp Ile Pro Phe Glu Gln Asp Glu Glu Ile Leu Arg Gly Arg Leu
   245 250 255
   Phe Phe Arg Arg Arg Val Ser Pro Glu Cys Gln Gln Leu Ile Glu Trp
   260 265 270
   Cys Leu Ser Leu Arg Pro Ser Glu Arg Pro Ser Leu Asp Gln Ile Ala
   275 280 285
   Ala His Pro Trp Met Leu Gly Thr Glu Gly Ser Val Pro Glu Asn Cys
   290 295 300
   Asp Leu Arg Leu Cys Ala Leu Asp Thr Asp Asp Gly Ala Ser Thr Thr
   305 310 315 320
   Ser Ser Ser Glu Ser Leu
   325
<210> 9
   <211> 1176
   <212> DNA
   <213> Homo sapiens
<400> 9
   cccacgcgtc cgcagagtgc cagcagctga tccggtggtg cctgtccctg cggccctcag 60
   agcggccgtc gctggatcag attgcggccc atccctggat gctgggggct gacgggggcg 120
   ccccggagag ctgtgacctg cggctgtgca ccctcgaccc tgatgacgtg gccagcacca 180
   cgtccagcag cgagagcttg tgaggagctg cacctgactg ggagctaggg gaccacctgc 240
   cttggccaga cctgggacgc ccccagaccc tgactttttc ctgcgtgggc cgtctcctcc 300
   tgcggaagca gtgacctctg acccctggtg accttcgctt tgagtgcctt ttgaacgctg 360
   gtcccgcggg acttggtttt ctcaagctct gtctgtccaa agacgctccg gtcgaggtcc 420
   cgcctgccct gggtggatac ttgaacccca gacgcccctc tgtgctgctg tgtccggagg 480
   cggccttccc atctgcctgc ccacccggag ctctttccgc cggcgcaggg tcccaagccc 540
   acctcccgcc ctcagtcctg cggtgtgcgt ctgggcacgt cctgcacaca caatgcaagt 600
   cctggcctcc gcgcccgccc gcccacgcga gccgtacccg ccgccaactc tgttatttat 660
   ggtgtgaccc cctggaggtg ccctcggccc accggggcta tttattgttt aatttatttg 720
   ttgaggttat ttcctctgag cagtctgcct ctcccaagcc ccaggggaca gtggggaggc 780
   aggggagggg gtggctgtgg tccagggacc ccaggccctg attcctgtgc ctggcgtctg 840
   tcctggcccc gcctgtcaga agatgaacat gtatagtggc taacttaagg ggagtgggtg 900
   accctgacac ttccaggcac tgtgcccagg gtttgggttt taaattattg actttgtaca 960
   gtctgcttgt gggctctgaa agctggggtg gggccagagc ctgagcgttt aatttattca 1020
   gtacctgtgt ttgtgtgaat gcggtgtgtg caggcatcgc agatgggggt tctttcagtt 1080
   caaaagtgag atgtctggag atcatatttt tttatacagg tatttcaatt aaaatgtttt 1140
   tgtacataaa aaaaaaaaaa aaaaaaaaaa aaaaaa 1176
<210> 10
   <211> 2447
   <212> DNA
   <213> Mus musculus
<400> 10
   gcagggcggg tgagagcgcc gtgaaagccg cggaacgccg tgcacctccg cgactctact 60
   ccggcaagct agtccggacg ggtcgtcgtc cccgcgcgcc accagccctt ggtgaaacga 120
   cagggagcgt ccggcttccc cagcaccgcc ctgcgagact caaaacagcc acaccgcaaa 180
   gcgagcctcg ggcggaagga ggcggagctt caggcggccc cgcctccgcg gaaggataca 240
   catctccgtg gtccaaaacc ccggggcgag gcggccgggg cgtgtgagct gctcggccag 300
   ctgccgtcta cgcgctttcg cgcggccacc gggcaactgc gccgcgcggc tgccccgctg 360
   agcgctcggc ctcggggccg tgggatccgc cgcgctgtct gcggtcagga agaccgccct 420
   cccgcgtcct tgccggacgg gtcagaggcg gcaccgcacg cgaggccacc cgcgatgctg 480
   ctgtccaagt tcggctccct ggcgcacctc tgcgggcctg gcggcgtgga ccacctccca 540
   gtgaagatcc tacagccagc caaggctgac aaggagagct tcgagaaggt gtaccaggtg 600
   ggcgccgtgc tgggcagcgg cggcttcggc acggtctacg cgggcagccg catcgccgac 660
   ggactcccgg tggctgtgaa gcacgtggtg aaggagcggg tgaccgagtg gggcagtctc 720
   ggcggagtgg ccgtgcccct ggaggtggtg ctgctgcgca aggtgggcgc ggcgggcggc 780
   gcgcgcggcg tcatccgctt gctggactgg ttcgagcggc ccgacggctt cttgttggtg 840
   ctggagcgac ccgagccggc acaggacctc ttcgacttca tcactgaacg aggcgccctg 900
   gacgagccgc tggcgcgtcg cttcttcgcg caggtgcttg ccgctgtgcg gcactgccac 960
   aattgtgggg tcgtgcaccg cgacatcaag gacgagaacc tgctggtgga cctgcgctcg 1020
   ggagagctga agctcatcga cttcggctcg ggcgcggtgc tcaaggacac ggtctacact 1080
   gactttgatg gcacccgtgt gtacagcccc ccagagtgga tccgatatca ccgatatcac 1140
   gggcggtctg ccactgtgtg gtctctgggt gtactgctct acgacatggt gtgtggggac 1200
   attccctttg agcaggatga ggagatcttg cgcggcaggc tctttttccg gaggagggtc 1260
   tccccagagt gccagcagct tattgagtgg tgtctctccc tgaggccctc agagaggccc 1320
   tccctggacc aaattgctgc ccacccctgg atgctgggga cagaggggag cgttccagag 1380
   aactgtgacc ttcggctttg tgccctggat actgacgacg gagccagtac cacttccagc 1440
   agtgagagct tgtgaggagg agaaggggcc tgggctcggc ctagccagcg ctctcccaga 1500
   attgaacact ttctgcctgg gatgtctgct gcaaaagcag tgacctctga cccctggtga 1560
   cctttgctct cggcaccggg cctgtttcct ttgctttgag tgcctttttg aacgctgctc 1620
   cacagggcct gggttttctt gagctcttct gtccaaagat ggctgagggc taagcaaggt 1680
   cctgccctgg gtggatactt gaaccagaga tcccgaccct gctgctccat ctcaggaggc 1740
   agccttcctg accaagtgtg tttgacatgg agcgccctgt ggtgcccacc tccaaccctc 1800
   cagtctcctg gtgttcatct gggcatgtct gcacaagcaa tgcaacgctg ggccactgct 1860
   gcccgtctgc ctccccggca cggcacggct ccgcacgcaa cctaagcgtg ccaccacggt 1920
   ctcttattta tggtgtgatc accctggagg gcgcccccgc cctgctgggg ctatttattg 1980
   tttaatttat ttgctgaggt tcctccaagc aaccaccttc tccaggcccc tggggtgttg 2040
   aaagtcaaat gtggctgttg agtccacaga cccccatcct aattcctgca cctggaggag 2100
   ttccccaacc cccgtgtttg cgggaggaag catttgtaca gtggctaatt taaggggagt 2160
   gggagaccct gtcaccctga gcactctgcg ctggggaggg gtttaaatta ttgaccttgt 2220
   acagtctgct tgctggctct gaaagctggg gttgggggac agagtctcaa gcccttaatt 2280
   tattttagca gctgtgtttc tgtgaccctg gtgtgactaa gcatcagggg tggggttgta 2340
   taagttcaaa agtgtgaaat gtctgaagat catatttttt atacaggtat ttcaattaaa 2400
   tgttttggta tataatggaa aaaaaaaaaa aaaaaaaaaa aaaaaaa                2447
<210> 11
   <211> 326
   <212> PRT
   <213> Mus musculus
<400> 11
   Met Leu Leu Ser Lys Phe Gly Ser Leu Ala His Leu Cys Gly Pro Gly
   1 5 10 15
   Gly Val Asp His Leu Pro Val Lys Ile Leu Gln Pro Ala Lys Ala Asp
   20 25 30
   Lys Glu Ser Phe Glu Lys Val Tyr Gln Val Gly Ala Val Leu Gly Ser
   35 40 45
   Gly Gly Phe Gly Thr Val Tyr Ala Gly Ser Arg Ile Ala Asp Gly Leu
   50 55 60
   Pro Val Ala Val Lys His Val Val Lys Glu Arg Val Thr Glu Trp Gly
   65 70 75 80
   Ser Leu Gly Gly Val Ala Val Pro Leu Glu Val Val Leu Leu Arg Lys
   85 90 95
   Val Gly Ala Ala Gly Gly Ala Arg Gly Val Ile Arg Leu Leu Asp Trp
   100 105 110
   Phe Glu Arg Pro Asp Gly Phe Leu Leu Val Leu Glu Arg Pro Glu Pro
   115 120 125
   Ala Gln Asp Leu Phe Asp Phe Ile Thr Glu Arg Gly Ala Leu Asp Glu
   130 135 140
   Pro Leu Ala Arg Arg Phe Phe Ala Gln Val Leu Ala Ala Val Arg His
   145 150 155 160
   Cys His Asn Cys Gly Val Val His Arg Asp Ile Lys Asp Glu Asn Leu
   165 170 175
   Leu Val Asp Leu Arg Ser Gly Glu Leu Lys Leu Ile Asp Phe Gly Ser
   180 185 190
   Gly Ala Val Leu Lys Asp Thr Val Tyr Thr Asp Phe Asp Gly Thr Arg
   195 200 205
   Val Tyr Ser Pro Pro Glu Trp Ile Arg Tyr His Arg Tyr His Gly Arg
   210 215 220
   Ser Ala Thr Val Trp Ser Leu Gly Val Leu Leu Tyr Asp Met Val Cys
   225 230 235 240
   Gly Asp Ile Pro Phe Glu Gln Asp Glu Glu Ile Leu Arg Gly Arg Leu
   245 250 255
   Phe Phe Arg Arg Arg Val Ser Pro Glu Cys Gln Gln Leu Ile Glu Trp
   260 265 270
   Cys Leu Ser Leu Arg Pro Ser Glu Arg Pro Ser Leu Asp Gln Ile Ala
   275 280 285
   Ala His Pro Trp Met Leu Gly Thr Glu Gly Ser Val Pro Glu Asn Cys
   290 295 300
   Asp Leu Arg Leu Cys Ala Leu Asp Thr Asp Asp Gly Ala Ser Thr Thr
   305 310 315 320
   Ser Ser Ser Glu Ser Leu
   325